# EUROPEAN PATENT SPECIFICATION

(11) **EP 0 711 271 B1**
(45) Date of publication and mention of the grant of the patent: **07.10.1998**
(21) Application number: 94921664.2
(22) Date of filing: 21.07.1994
(51) Int. Cl.: C07C 211/42, C07C 211/49, C07C 217/52, C07C 323/23, A61K 31/135

(54) **AMINOINDANES AND RELATED COMPOUNDS USEFUL AS CALCIUM-CHANNEL ANTAGONISTS**
AMINOINDANE UND VERWANDTE VERBINDUNGEN ALS CALCIUMKANAL-ANTAGONISTEN
AMINOINDANES ET COMPOSES APPARENTES UTILES COMME ANTAGONISTES DES CANAUX A CALCIUM

(30) Priority: 28.07.1993 GB 9315600
(43) Date of publication of application: 15.05.1996
(73) Proprietor: SMITHKLINE BEECHAM PLC, Brentford, Middlesex TW8 9EP (GB)
(72) Inventor: HARLING, John, David, The Pinnacles Harlow Essex CM 19 5AD (GB); ORLEK, Barry, Sidney, The Pinnacles Harlow Essex CM 19 5AD (GB)
(74) Representative: Florence, Julia Anne
(86) International application number: EP9402410
(87) International publication number: WO9504027

(56) References cited:
- EP-A- 0 303 961
- EP-A- 0 371 508
- DE-A- 2 229 359
- US-A- 4 652 561

## Description

The present invention relates to carbocyclic derivatives, processes for their preparation, pharmaceutical compositions containing them and their use in therapy, in particular as calcium channel antagonists, e.g. for the treatment of ischaemic stroke.

Stroke is reportedly the third most common cause of death in the developed world. Current therapies for ischaemic stroke are limited and have a number of disadvantages, such as the risk of exacerbating haemorrhage. There is therefore a need for new and improved treatments for ischaemic stroke.

EPA 303961 describes compounds of the formula 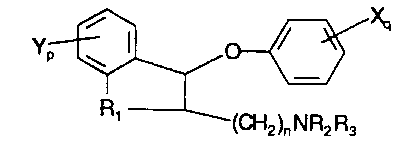 wherein R₁ is C₁₋₃ alkylene, n and p are each *inter alia* zero, R₂ and R₃ represent *inter alia* hydrogen or lower alkyl, X is *inter alia* lower alkyl, lower alkoxy, CF₃ or halogen and q is zero, 1 or 2. The compounds are said to be useful as antidepressants and as inhibitors of synaptic norepinephrine and serotonin uptake.

EPA 371508 describes similar compounds of the formula: 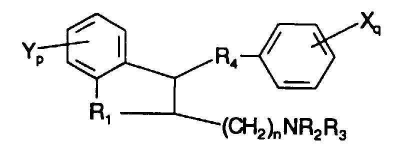 wherein R₁, R₂, R₃, n, p, q and X may have the values recited hereinabove and R₄ is oxy or thio, which compounds are said to be useful for the treatment of drug-resistant malaria and other drug-resistant protozoal infections.

US-A-4,652,561 discloses, compounds of the formula 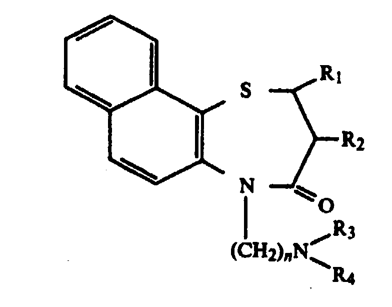 wherein R₁ is phenyl substituted with 1 to 3 lower alkoxy groups or 1 to 3 halogens; R₂ is hydroxy, lower alkoxy, lower alkanoyloxy, lower cycloalkylcarbonyloxy; 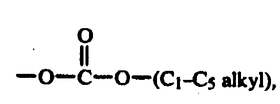 or 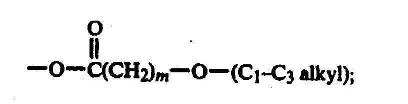 R₃ and R₄ are independently lower alkyl, phenyl lower alkyl or together form a piperidine or pyrrolidine ring; is 2 to 4; m is 1 to 2; or pharmaceutically acceptable acid addition salts thereof are described. These compounds have activity as calcium channel blockers and accordingly, are useful as agents for lowering blood pressure, and as agents for treating ischemia.

We have now found that certain carbocyclic derivatives exhibit activity as calcium channel antagonists.

The present invention therefore provides in a first aspect compounds of formula (I) : 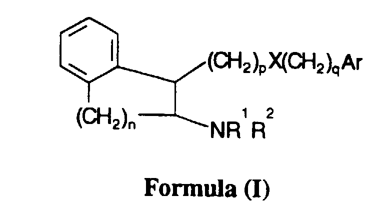 wherein
X represents O, S, NH or a bond;
p and q independently represent 0-4 such that the sum of p+q is at least 1;
R¹ and R² each independently represent hydrogen, C₁₋₆alkyl, C₃₋₆cycloalkyl, or C₁₋₄alkyl-C₃_₆cycloalkyl;
n is 1,2 or 3; and
Ar represents phenyl optionally substituted by 1 to 3 substituents selected from :
   halo, C₁₋₄alkyl, C₁₋₄alkoxy, C₁₋₂alkylenedioxy, trifluoromethyl, trifluoromethoxy, CN, NO₂, amino, mono- or di- alkylamino, optionally substituted benzoyl and Ph(CH₂)ᵣY(CH₂)ₛ- where Ph is optionally substituted phenyl, Y is oxygen or a bond and r and s each independently represent 0-4 provided that the sum of r+s is not greater than 4, or
   Ar represents an optionally substituted unsaturated monocyclic heteroaryl ring system containing 5 or 6 ring members, or an optionally substituted, unsaturated or partially saturated bicyclic aryl or heteroaryl ring system containing 8-10 ring members, and salts thereof.

Preferably R¹ is methyl, cyclopropylmethyl, isopropyl or hydrogen, particularly preferably methyl.

Preferably R² is hydrogen or methyl, particularly preferably hydrogen, and NR¹R² is preferably amino, methylamino or isopropylamino.

Preferably X is a bond, and preferably the sum of p and q is one.

In the compounds of formula (I) when Ar represents phenyl this is advantageously substituted by optionally substituted benzoyl or a group Ph(CH₂)ᵣY(CH₂)ₛ-. Preferably r and s independently represent zero or 1, such that the sum of r and s does not exceed 1. Particularly preferred substituents of the formula Ph(CH₂)ᵣY(CH₂)ₛ- thus include optionally substituted benzyloxy, benzyl and phenoxy.

Suitable substituents for the group Ph and for benzoyl include halogen, especially fluorine, chlorine and bromine, C₁₋₄alkoxy especially methoxy, C₁₋₄alkyl especially methyl, trifluoromethyl and trifluoromethoxy, and particularly 3-chloro, 3-fluoro, 4-chloro, 4-fluoro-, and 3,4-dichloro. 4-Fluoro is a particularly preferred substituent because this blocks metabolic hydroxylation of the phenyl group.

When Ar represents a bicyclic aryl suitable groups include naphthyl.

When Ar represents unsaturated monocyclic heteroaryl suitable groups include optionally substituted pyridyl, thienyl and furyl, e.g. optionally substitued 2-pyridyl, 3-pyridyl, 4-pyridyl, 2-thienyl and 2-furyl, and in particular 5-chloro-2-thienyl.

When Ar represents bicyclic heteroaryl, suitable groups include benzofuranyl e.g. 5-benzo[b]furanyl.

Suitable substituents for bicyclic aryl and monocyclic and bicyclic heteroaryl groups include halogen e.g. chlorine or bromine, C₁₋₄alkyl e.g. methyl, C₁₋₄alkoxy e.g. methoxy, trifluoromethyl, trifluoromethoxy, phenyl, phenylC₁₋₄alkyl e.g. 2-phenylethyl, and phenyl C₁₋₄alkoxy e.g. benzyloxy.

Particular meanings of Ar include phenyl substituted by benzyl, benzyloxy, phenoxy or benzoyl.

Preferred meanings of Ar include 4-benzylphenyl, 4-benzyloxyphenyl, 4-benzoylphenyl, 4-(4-fluorobenzoyl)phenyl, 4-phenoxyphenyl, 4-(4-fluorophenoxy)phenyl, 3,4-dichlorophenyl, 4-trifluoromethylphenyl, 4-cyclopropylmethoxyphenyl and 4-n-butyloxyphenyl.

Alkyl groups present in the compounds of formula (I), alone or as part of another group, can be straight or branched. Thus, a C₁₋₆dkyl group may be for example methyl, ethyl, n-propyl, n-butyl,n-pentyl, n-hexyl or any branched isomer thereof such as isopropyl or t-butyl. Preferred meanings of C₃₋₆cycloalkyl are cyclopropyl and cyclohexyl, and preferred meanings of C₁₋₄alkyl-C₃₋₆cycloalkyl are cyclopropylmethyl and cyclohexylmethyl.

It will be appreciated that for use in medicine a salt of a compound (I) should be pharmaceutically acceptable. Examples of pharmaceutically acceptable salts include inorganic and organic acid addition salts such as hydrochloride, hydrobromide, sulphate, phosphate, acetate, fumarate, maleate, citrate, lactate, tartrate, oxalate, methanesulphonate or similar pharmaceutically acceptable inorganic or organic acid addition salts. Other non-pharmaceutically acceptable salts may be used for example in the isolation of final products and are included within the scope of this invention.

It will be appreciated that the compounds of formula (I) contain two or more asymmetric centres. Such compounds will exist as optical isomers (enantiomers). Both the pure enantiomers, racemic mixtures (50% of each enantiomer) and unequal mixtures of the two are included within the scope of the invention. Further, all diastereomeric forms possible (pure enantiomers and mixtures thereof) are within the scope of the invention. In particular it will be appreciated that the substituents on the carbocyclic (e.g. indane) nucleus may both lie on the same side with respect to the plane of the ring *(cis*-configuration) or on opposite sides (*trans*-configuration). Both forms and all mixtures thereof are included within the scope of this invention. Preferably the compounds of formula (I) are of the *cis*-configuration.

In accordance with convention the (+) and (-) designations used herein indicate the direction of rotation of plane-polarised light by the compounds. The prefix (+) indicates that the isomer is dextrorotatory (which can also be designated d) and the prefix (-) indicates the levorotatory isomer (which can also be designated 1).

Particular preferred compounds of the formula (I) include :
(±)*trans*-1-(4-Benzylbenzyl)-2-methylaminoindane;
(±)*trans*-1-(4-Benzylbenzyl)-2-dimethylaminoindane;
(±)*trans*-1-(4-Benzyloxybenzyl)-2-methylaminoindane;
(±)*trans*-1-(4-Benzyloxybenzyl)-2-dimethylaminoindane;
(±)*trans*-1-(4-Benzyloxybenzyl)-2-ethylaminoindane;
(±)*cis*-1-(4-Benzyloxybenzyl)-2-aminoindane;
(±)*cis*-1-(4-Benzyloxybenzyl)-2-methylaminoindane;
(±)*cis*-1-[3-(4-Benzyloxyphenyl)propyl]-2-aminoindane;
(±)*cis*-1-[3-(4-Benzyloxyphenyl)propyl]-2-methylaminoindane;
(±)*trans*-1-[(4-Benzyl)benzyl]-2-aminoindane;
(±)*cis*-1-[(4-Benzyl)phenoxymethyl]-2-aminoindane;
(±)*cis*-1-[(4-Benzyl)phenoxymethyl]-2-methylaminoindane;
(±)*cis*-1-(4-Phenoxybenzyl)-2-methylaminoindane;
(±)*cis*-1-(3,4-Dichlorobenzyl)-2-aminoindane;
(±)*cis*-1(3,4-Dichlorobenzyl)-2-methylaminoindane;
(±)*cis*-1-(4-Benzyioxybenzyl)-2-methylaminoindane;
(-)*cis*-1-(4-Benzyloxybenzyl)-2-methylaminoindane;
(±)*cis*-1-(4-Trifluoromethylbenzyl)-2-aminoindane;
(±)*cis*-1-(4-PhenoxybenzylY2-aminoindane;
(±)*cis*-1-[(4-Benzoyl)phenoxymethyl]-2-aminoindane;
(±)*cis*-1-(4-Benzyloxybenzyl)-2-dimethylaminoindane;
(±)*cis*-1-(4-Cyclopropylmethoxybenzyl)-2-aminoindane;
(±)*cis*-1-(3,4-Dichlorophenoxymethyl)-2-aminoindane;
(±)*cis*-1-(4-n-Butoxybenzyl)-2-aminoindane;
(±)*cis*-2-Amino-1-[(5-chloro-2-thienyl)methyl]indane;
(±)*cis*-1-[(5-Chloro-2-thienyl)methyl]-2-methylaminoindane;
(±)*cis*-2-Cyclopropymethylamino-1-(3,4-dichlorobenzyl)-indane;
(±)*cis*-1-(3,4-Dichlorobenzyl)-2-dimethylaminoindane;
(±)*cis*-1-(3,4-Dichlorobenzyl)-2-isopropylaminoindane;
(±)*cis*-1-[4-(4-Fluorophenoxy)benzyl]-2-aminoindane;
(±)*cis*-1-(4-(4-Fluorophenoxy)benzyl]-2.methylaminoindane;
(±)*cis*-1-[4-(4-Fluorophenoxy)benzyl]-2-isopropylaminoindane;
(±)*cis*-1-(4-Benzoylbenzyl)-2-aminoindane;
(±)*cis*-1-(4-Trifluoromethylbenzyl)-2-methylaminoindane;
and salts thereof.

The compounds of the present invention can be prepared by processes analogous to those known in the art. The present invention therefore provides in a further aspect, a process for the preparation of compounds of formula (I) which comprises :
(a) to prepare a compound of formula (I) wherein R² is methyl, reduction of a compound of formula (II) 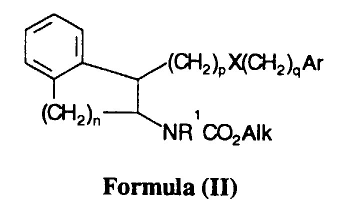 wherein n, p, q, R¹, Ar and X are as hereinbefore defined and Alk is a C₁-₄alkyl group;
(b) to prepare a compound of formula (I) wherein at least one of R¹ and R² is hydrogen, deprotection of a compound of formula (II);
(c) to prepare a compound of formula (I) wherein R¹ and R² are both hydrogen and p is 1 to 4, reduction of a compound of formula (III): 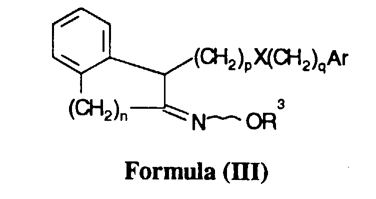 wherein n, p, q, X and Ar are as hereinbefore defined and R³ is hydrogen, C₁₋₄alkyl or phenylC₁₋₄alkyl (e.g. benzyl);
(d) to prepare a compound wherein X is O, S or NH reaction of a compound of formula (IV) 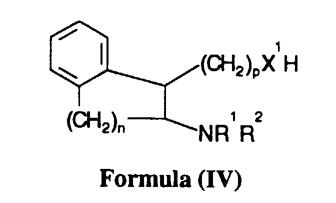 wherein R¹, R², p and n are as hereinbefore defined and X¹ is O, S or NH, with a compound of formula L(CH₂)_{q}Ar wherein L is a leaving group and q and Ar are as hereinbefore defined;
(e) to prepare a compound wherein X is O, S or NH, reaction of a compound formula (V): 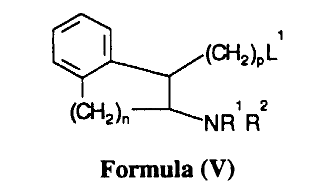 wherein R¹, R², p and n are as hereinbefore defined and L¹ is a group displaceable by a nucleophile, with a compound HX(CH₂)_{q}Ar wherein X, q and Ar are as hereinbefore defined;
(f) interconversion of a compound of formula (I) to a different compound of formula (I), e.g.
   (i) where one of R¹ and R² is hydrogen and the other is alkyl, conversion to a compound of formula (I) wherein R¹ and R² are both alkyl, or
   (ii) where R¹ and R² are both hydrogen, conversion to a compound of formula (I) where at least one of R¹ and R² represent alkyl;
   (iii) conversion of a benzoyl substituent in the group Ar to benzyl;
   and optionally after any of the above processes, forming a salt of formula (I).

Reduction according to process (a) may be effected using a suitable reducing agent such as lithium aluminium hydride, preferably in an inert solvent such as tetrahydrofuran or diethyl ether.

Deprotection according to process (b) may be carried out by conventional methods. Thus for example an ethoxycarbonyl group may be removed by hydrolysis under basic conditions. A *tert*-butoxycarbonyl group may be cleaved using trifluoroacetic acid, in a solvent such as dichloromethane.

Reduction of a compound of formula (III) according to process (c) may be effected using a reducing agent such as lithium aluminium hydride or NaBH₃(OCOCF₃) in an inert solvent such as an ether, e.g. diethyl ether or tetrahydrofuran. In general this process gives predominantly the *cis*-form of the product. Preferably in process (c) R³ is C₁₋₄alkyl.

In process (d) the reaction between a compound of formula (IV) and L(CH₂)_{q}Ar may be effected under conditions which depend on the nature of L and the value of q. For example when q is zero, L is preferably fluoro and the reaction is preferably effected in the presence of a strong base such as sodium hydride, and in a polar organic solvent such as dimethylsulphoxide or dimethylformamide. In this case the aryl group is preferably substituted by an activating group such as benzoyl or CF₃. When q is other than zero, L may be for example halo or preferably a sulphonic acid residue such as a tosylate or mesylate and the reaction may be carried out using standard conditions, in a solvent and optionally in the presence of a base, which solvent and base may, if desired be selected from those described above. If necessary during process (d) the nitrogen atom may be protected by methods well known in the art, e.g. a carbamate which may be removed for example as described in process (b).

The reaction between a compound of formula (V) and HX(CH₂)_{q}Ar in process (e) can take place under conditions which depend on the nature of L¹ and X. For example when L¹ is hydroxy, X is oxygen or sulphur, and q is 0, the reaction is carried out in the presence of diethyl azodicarboxylate and triphenyl phosphine. Such a reaction is known as the Mitsunobu reaction (as described in Synthesis 1981, 1; and J. Org. Chem. **1991**, 56, 670-672). Alternatively the leaving group L¹ may be for example a halogen atom or a sulphonyloxy group eg. methane-sulphonyloxy or p-toluene sulphonyloxy in which case the reaction may be effected using standard conditions, in the presence or absence of solvent, optionally in the presence of a base.

Interconversions according to process (f) may be effected by alkylation of a compound (I) wherein one of R¹ and R² is hydrogen and the other is alkyl or where R¹ and R² are both hydrogen, using an appropriate alkylating agent such as an alkyl halide e.g. an alkyl bromide or iodide, in the presence of a base, such as potassium carbonate. The reaction may be carried out in a suitable solvent such as acetone. Alternatively said compound of formula (I) may first he acylated, using for example an alkylhaloformate such as ethyl chloroformate, preferably in the presence of a tertiary amine such as triethylamine, or a carbonate such as di-*tert*-butyldicarbonate, in the presence of sodium hydroxide and a solvent such as dioxane, to provide a compound of formula (II) followed by reduction as described above. In a further method a compound of formula (I) may be subjected to reductive alkylation using an appropriate aldehyde (e.g. formaldehyde) or ketone, and a reducing agent such as sodium cyanoborohydride. Reduction of a benzoyl substituent to benzyl may be effected using e.g. sodium borohydride in trifluoroacetic acid.

It will be appreciated that when any of the processes described herein involve a reduction step it will generally be desirable to employ reducing agents and conditions which do not affect or disturb substituents which are intended to be retained in the final product. The choice of appropriate reducing agents and conditions will be readily apparent to the skilled practitioner. Thus for example when Ar represents 3,4-dichlorophenyl it is preferable to avoid the use of lithium aluminium hydride under forcing (e.g. reflux) conditions.

Processes (a), (b), (d) and (f) generally proceed with retention of the *cis* or *trans* configuration of the starting material. Thus the stereochemistry of the final product is usually determined by the configuration of formula (II) which in turn is governed by its method of preparation.

A *trans*-isomer of formula (II) wherein R¹ is hydrogen and Alk is *tert*-butyl may be prepared by reaction of a compound of formula (VI): 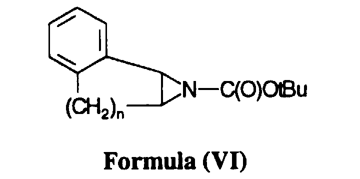 with a Grignard derivative of formula L²Mg(CH₂)ₚX(CH₂)_{q}Ar, wherein L² is halo. In this reaction X is preferably a bond. The reaction is conveniently effected in the presence of copper (I) bromide dimethylsulphide complex and in a solvent such as toluene. The Grignard derivative may be prepared using standard procedures from a corresponding compound of formula L²(CH₂)ₚX(CH₂)_{q}Ar, optionally *in situ.* Compounds of formula L²(CH₂)ₚX(CH₂)_{q}Ar are commercially available or may be prepared using standard methods; for example a phenol such as 4-fluorophenol is reacted with 4-fluorobenzaldehyde under basic conditions, and the substituted benzaldehyde can be reduced, e.g. with sodium borohydride, and the alcohol appropriately activated, e.g. by conversion to the benzyl chloride with thionyl chloride. The reaction with 4-fluorophenol can be conveniently carried out using potassium carbonate in a dipolar aprotic solvent such as dimethyl sulphoxide in the presence of a suitable Crown ether such as 18-Crown-6.

Cis-isomers of formula (II) may be prepared by acylation of a corresponding compound of formula (I) as described in process (f) above.

Compounds of formula (II) (both cis and trans forms) wherein R¹ is alkyl may be prepared by alkylation of formula (II) wherein R¹ is hydrogen for example using an alkyl halide in the presence of a base such as sodium hydride and in a suitable solvent e.g. dimethylformamide. Alternatively a compound (II) wherein R¹ is hydrogen may be reduced as described in process (a) above and subsequently acylated as described for process (f) above.

A compound of formula (VI) may be prepared by cyclisation of a compound of formula (VII) : 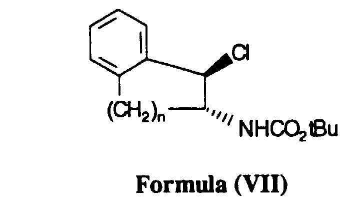 using sodium hydride in a suitable solvent for example an ether such as tetrahydrofuran optionally containing a crown ether such as 15-crown-5; or dimethyl formamide.

Compounds of formula (VII) may be prepared by addition of N,N-dichloro-t-butylcarbamate to an olefin of formula (VIII):

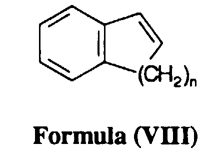

A compound of formula (m) may be prepared by reaction of a compound of formula (IX): 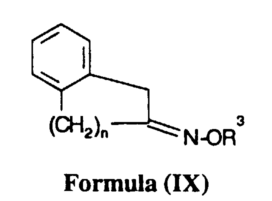 with a compound of formula L²(CH₂)ₚX(CH₂)_{q}Ar in the presence of lithium bis-(trimethylsilyl)amide in a solvent such as tetrahydrofuran, followed if necessary by interconversion of Ar groups: for example compounds of formula (III) in which Ar is phenyl substituted by benzyloxy can be reduced to the compounds in which Ar is phenyl substituted by hydroxy (e.g. using palladium on charcoal and ammonium formate in a solvent such as methanol) and these compounds can be alkylated e.g. with an alkyl bromide and potassium carbonate. The compounds of formula L²(CH₂)ₚX(CH₂)_{q}Ar can be prepared as described above.

*Trans*-isomers of formula (IV) wherein X¹ is oxygen and p is zero may be prepared as described in the literature e.g. Chem. Pharm. Bull. 1977, **25**, 1060. Corresponding *cis*-isomers may be obtained by oxidation of the *trans*-isomer using Jones reagent to give a ketone followed by reduction with lithium tri-O-isobutyl borohydride (L-Selectride®, Aldrich). Reduction of the ketone using sodium borohydride regenerates the trans-isomer.

Compounds of formula (IV) wherein X¹ is S or NH may be prepared from the corresponding sulphonyloxy compound e.g. a mesylate and an appropriate amino or thiol reagent using standard methods. The sulphonyloxy derivative may itself be prepared from the corresponding alcohol in conventional manner.

Compounds of formula (V) wherein L¹ is OH and p is zero may be prepared as described for the compounds of formula (IV). When L¹ is halogen or a sulphonyloxy group such compounds may be prepared from the corresponding alcohol in conventional manner.

When a compound of formula (I) is obtained as a mixture of enantiomers, these may be separated by conventional methods such as crystallisation in the presence of a resolving agent, or chromatography, for example using a chiral HPLC column.

Preferably the compounds of formula (I) are resolved by conversion to a mixture of diastereomeric amides, which are separated by conventional methods and then reconverted into resolved compounds of formula (I). Preferably the amides are formed with S(+)-α-methoxyphenylacetic acid, and the separated amides can be converted into the resolved amines by treatment with an excess of methyllithium, or by treatment with an excess of potassium t-butoxide in wet tetrahydrofuran as generally described in U.S. 5,149,714.

Compounds of formula (I) have been found to exhibit high calcium influx blocking activity for example in neurons. As such the compounds are expected to be of use in therapy in treating conditions and diseases related to an accumulation of calcium in the brain cells of mammals, in particular humans. For example, the compounds are expected to be of use in the treatment of anoxia, ischaemia including for example stroke, migraine, visceral pain, epilepsy, traumatic head injury, AIDS-related dementia, neurodegenerative diseases such as Alzheimer's disease and age-related memory disorders, mood disorders and drug addiction withdrawal such as ethanol addiction withdrawal.

In a further aspect of the invention there is therefore provided a method of treatment of conditions or diseases related to (e.g. caused or exacerbated by) the accumulation of calcium in the brain cells of mammals which comprises administering to a subject in need thereof an effective amount of a compound of formula (I) or a pharmaceutically acceptable salt thereof. Thus, for example, the present invention provides a method of treatment of anoxia, ischaemia including for example stroke, migraine, visceral pain, epilepsy, traumatic head injury, AIDS-related dementia, neurodegenerative diseases such as Alzheimer's disease, and age-related memory disorders, mood disorders and drug addiction withdrawal such as ethanol addiction withdrawal, which comprises administering to a subject in need thereof, an effective amount of a compound of formula (I) or a pharmaceutically acceptable salt thereof.

The present invention also provides the use of a compound of formula (I) or a pharmaceutically acceptable salt thereof in the manufacture of a medicament for the treatment of a condition or disease related to the accumulation of calcium in the brain cells of a mammal.

For use in medicine, the compounds of formula (I) are usually administered in a standard pharmaceutical composition. The present invention therefore provides in a further aspect pharmaceutical compositions comprising a novel compound of formula (I) or a pharmaceutically acceptable salt thereof and a pharmaceutically acceptable carrier or excipient.

The compounds of formula (I) may be administered by any convenient method for example by oral, parenteral, buccal, rectal or transdermal administration and the pharmaceutical compositions adapted accordingly.

The compounds of formula (I) and their pharmaceutically acceptable salts which are active when given orally can be formulated as liquids or solids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Compounds of the invention may also be administered parenterally, by bolus injection or continuous infusion. Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Both liquid and solid compositions may contain other excipients known in the pharmaceutical art, such as cyclodextrins.

Preferably the composition is in unit dose form such as a tablet, capsule or ampoule.

Each dosage unit for oral administration contains preferably from 1 to 250 mg (and for parenteral administration contains preferably from 0.1 to 60 mg) of a compound of the formula (I) or a pharmaceutically acceptable salt thereof calculated as the free base.

The daily dosage regimen for an adult patient may be, for example, an oral dose of between 1 mg and 500 mg, preferably between 1 mg and 250 mg, e.g. 5 to 200 mg or an intravenous, subcutaneous, or intramuscular dose of between 0.1 mg and 100 mg, preferably between 0.1 mg and 60 mg, e.g. 1 to 40 mg of the compound of the formula (I) or a pharmaceutically acceptable salt thereof calculated as the free base, the compound being administered 1 to 4 times per day. Alternatively the compounds of the invention may be administered by continuous intravenous infusion, preferably at a dose of up to 400 mg per day. Thus, the total daily dosage by oral administration will be in the range 1 to 2000 mg and the total daily dosage by parenteral administration will be in the range 0.1 to 400 mg. Suitably the compounds will be administered for a period of continuous therapy, for example for a week or more.

### BIOLOGICAL DATA

### Ca²⁺ Current Measurement

### Cell preparations

Sensory neurons from dorsal root ganglia were dissociated from 1 day old rat pups (Forda et al, Developmental Brain Research, 22 (1985), 55-65). Cells were plated out onto glass coverslips and used within 3 days to permit effective voltage clamp of Ca²⁺ currents.

Superior cervical ganglion neurons were isolated and cultured following a method modified from Marrion *et al,* Neurosci. Lett., 77, 55-60 (1987). Cells were plated onto laminin coated plastic tissue culture dishes and incubated at 37°C until just prior to recording. Electrophysiological recordings were performed from 2 to 9 days after dissociation.

### Solutions

The pipette (internal solution) contained in mM: CsCl, 130; HEPES, 10; EGTA, 10; MgCl₂, 4; ATP, 2; buffered to pH 7.2 with CsOH. Cells were bathed in a normal Tyrodes solution before establishment of whole cell recording when the bathing solution was changed to one allowing isolation of Ca²⁺ currents. The external solution for recording Ca²⁺channel currents contained in mM: BaCl₂, 10; TEA-Cl, 130; glucose, 10; HEPES, 10; MgCl₂, 1; buffered to pH 7.3 with TEA-OH. Barium was used as the charge carrier as this assists in current isolation and calcium dependent inactivation of current is avoided. Compounds were dissolved in DMSO to make a 20mM stock solution. At the highest drug concentration used the vehicle (0.1%) had no significant effect on Ca²⁺ currents. All experiments were performed at 21 to 24°C. Whole cell currents were recorded using List EPC-7 amplifiers and stored, digitised for later analysis using PC based software similar to that described previously (Benham & Tsien, Journal of Physiology (1988), **404**, 767-784).

### Results

### Ca²⁺ currents

Peak voltage gated Ca²⁺ channel currents of up to 10 nA were recorded using 10mM Ba²⁺ as charge carrier. Currents were evoked from a holding potential of -80mV to a test potential of 0 or +10mV every 15 seconds. This test potential was at the peak of the current voltage relationship and assessing block at this point reduced any errors due to drifting holding potential. Some cells showed slow rundown of current as is commonly seen when recording Ca²⁺ currents. The rundown rate was measured in control conditions and extrapolated through the time of drug application to derive a rundown corrected control value.

### Dorsal Root Ganglion Cells

Block by 20 µM drug was assessed 3 minutes after drug application. Compounds of the invention described in the specific examples gave percentage inhibition of plateau Ca²⁺ current in the range 40-100%.

### Superior Cervical Ganglion Cells

Once a constant calcium current had been recorded for 4 successive pulses (1 minute) 10 µM Nimodipine, a dihydropyridine, was applied to the cell to block L type calcium current. After three minutes 5µM drug was coapplied with 10µM Nimodipine for three minutes. Such drug application tested the block of the remaining, predominantly N type, calcium current.

Compounds of the invention gave percentage inhibition of plateau Ca²⁺ current in the range 7 to 97% at 5µM.

### PHARMACEUTICAL FORMULATIONS

The following represent typical pharmaceutical formulations according to the present invention, which may be prepared using standard methods.

| **IV Infusion** | |
|---|---|
| Compound of formula (I) | 1-40 mg |
| Buffer | to pH ca 7 |
| Solvent/complexing agent | to 100 ml |

| **Bolus Injection** | |
|---|---|
| Compound of formula (I) | 1-40 mg |
| Buffer | to pH ca 7 |
| Co-Solvent | to 5 ml |

- Buffer:: Suitable buffers include citrate, phosphate, sodium hydroxide/hydrochloric acid.
- Solvent :: Typically water but may also include cyclodextrins (1-100 mg) and co-solvents such as propylene glycol, polyethylene glycol and alcohol.

| **Tablet** | |
|---|---|
| Compound | 1 - 40 mg |
| Diluent/Filler * | 50 - 250 mg |
| Binder | 5 - 25 mg |
| Disentegrant * | 5 - 50 mg |
| Lubricant | 1 - 5 mg |
| Cyclodextrin | 1 - 100 mg |

| | |
|---|---|
| * may also include cyclodextrins | |

- Diluent :: e.g. Microcrystalline cellulose, lactose, starch
- Binder :: e.g. Polyvinylpyrrolidone, hydroxypropymethylcellulose
- Disintegrant :: e.g. Sodium starch glycollate, crospovidone
- Lubricant:: e.g. Magnesium stearate, sodium stearyl fumarate.

| **Oral Suspension** | |
|---|---|
| Compound | 1 - 40 mg |
| Suspending Agent | 0.1 - 10 mg |
| Diluent | 20 - 60 mg |
| Preservative | 0.01 - 1.0 mg |
| Buffer | to pH ca 5 - 8 |
| Co-solvent | 0 - 40 mg |
| Flavour | 0.01 - 1.0 mg |
| Colourant | 0.001 - 0.1 mg |
| Suspending agent : | e.g. Xanthan gum, microcrystalline cellulose |
| Diluent : | e.g. sorbitol solution, typically water |
| Preservative : | e.g. sodiuim benzoate |
| Buffer : | e.g. citrate |
| Co-solvent : | e.g. alcohol, propylene glycol, polyethylene glycol, cyclodextrin |

The invention is further illustrated by the following non-limiting Preparations and Examples:

### Preparation 1

### (±)trans-1-Chloro-2-tert-butoxycarbonylaminoindane

To a solution of indene (32.58g, 0.28 mol) in toluene (350 ml) under nitrogen was added dropwise a solution of N,N-dichloro-*tert*-butylcarbamate (60g, 0.323 mol) in toluene (200 ml). The solution was then stirred at 50°C for 5 h. Saturated aqueous sodium metabisulfite (500 ml) was then added with cooling from an external ice/water bath. The two phase mixture was then vigorously stirred at room temperature for 3 h. The phases were then separated and the aqueous phase extracted with diethyl ether (3 x 100 ml). The organic phases were then combined and washed successively with saturated aqueous sodium bicarbonate, water and brine. After drying over Na₂SO₄, solvents were removed *in vacuo* to afford the **title compound** as a white solid (71.60g).
¹H Nmr (CDCl₃) δ: 1.47 (9H, s), 2.80 (1H, dd, J=7,15Hz), 3.52 (1H, dd, J=7,15Hz), 4.47 (1H, m), 4.78 (1H, m), 5.19 (1H, br. s), 7.27 (3H, m),7.42 (1H, m).

### Preparation 2

### (±) N-tert-Butoxycarbonyl-1,2-iminoindane

To a suspension of NaH (80% disp. in oil, 2.016g, 67.2 mmol) in dry tetrahydrofuran (300 ml) was added (±) *trans*-1-chloro-2-tert-butoxycarbonylaminoindane (15g, 56 mmol) and 15-crown-5 (50µl). The mixture was then warmed at 50°C under nitrogen for 18 h after which it was poured into water (700ml) and extracted with diethyl ether (3x150ml). The combined organic extracts were dried (Na₂SO₄) and solvents were removed *in vacuo* to afford the **title compound** as a brown solid (12.67g).
¹H Nmr (CDCl₃) δ: 1.08 (9H, s), 3.04 (1H, dd, J=5,18Hz),3.50 (2H,m), 3.84 (1H, m), 7.20 (3H, d, J=4Hz), 7.46 (1H, m).

### Preparation 3

### (±) trans-1-Benzyl-2-tert-butoxycarbonylaminoindane

To a mixture of (±) N-*tert*-butoxycarbonyl-1,2-iminoindane (1.5g, 6.5mmol) and copper(I) bromide dimethylsulfide complex (267mg, 1.3mmol) in dry toluene (45ml) at -30°C under nitrogen was added a solution of benzyl magnesium bromide (Aldrich)(lM in diethyl ether, 6.5ml, 6.5mmol). After stirring at -30°C for 40 minutes the reaction was quenched with water. The layers were separated and the aqueous layer extracted with diethyl ether (3x25ml). The combined organic phases were dried over sodium sulfate and concentrated in *vacuo.The* residue was subjected to column chromatography on silica gel eluting with 15% diethyl ether in hexanes to afford the **title compound** as a pale yellow solid (1.77g).
¹H Nmr (CDCl₃) δ: 1.40 (9H, s), 2.69 (1H, dd, J=5, 17Hz), 2.91 (1H, dd, J = 7, 15Hz), 3.03 (1H, dd, J=7, 15Hz), 3.24 (2H, m), 4.18 (1H, br. s), 4.58 (1H, br. s), 6.88 (1H, d, J=6Hz), 7.04-7.32 (8H, m).

### Preparation 4

### (±) trans-1-Benzyl-2-(N-methyl-N-tert-butoxycarbonylamino)indane

To a suspension of sodium hydride (80% disp. in oil, 135mg, 4.5mmol) in dry N,N-dimethylformamide (2ml) under nitrogen was added dropwise a solution of (±) *trans*-1-benzyl-2-*tert*-butoxycarbonylaminoindane (1.33g, 4.1 mmol) in N, N-dimethylformamide (8ml). After stirring the mixture for 30 minutes at room temperature, iodomethane (280µl, 4.5mmol) was added dropwise and stirring continued for a further 1 h. The reaction mixture was then poured into a large excess of sat. ammonium chloride and extracted with diethyl ether (3 x 40ml). The combined organic extracts were dried over sodium sulfate and concentrated *in vacuo.* The residue was subjected to column chromatography on silica gel eluting with 10% diethyl ether in hexanes to afford the **title compound** as a colourless oil (1.14g).
¹H Nmr (CDCl₃) δ: 1.42 (9H, s), 2.58 (3H, br. s), 2.78-3.14 (4H, m), 3.53 (1H, q, J= 7Hz), 4.61+4.84 (1H, br. s + br. s, rotomers), 6.96-7.33 (9H, m).

### Preparation 5

### (±) trans-1-(2-Benzylbenzyl)-2-tert-butoxycarbonylaminoindane

2-Benzylbenzyl chloride (2.15g, 10mmol) was dissolved in dry diethyl ether (10 ml) and a small amount of this solution added to dried magnesium turnings (243mg, 10 mmol). The reaction was initiated with a crystal of iodine before the remainder of the solution of 2-benzylbenzyl chloride was added at such a rate to maintain gentle reflux in the exothermic reaction. After stirring at room temperature for a further 45 minutes this solution was added in one portion to a mixture of (±) N-*tert*-butoxycarbonyl-1,2-iminoindane (2.31g, 10mmol) and copper (I) bromide dimethylsulfide complex (411mg, 2 mmol) in toluene (70ml) at -30°C under nitrogen. After stirring at -30°C for 40 minutes the reaction was quenched with sat. aqueous ammonium chloride. The layers were separated and the aqueous layer extracted with diethyl ether (3x50ml). The combined organic phases were dried over sodium sulfate and concentrated *in vacuo.* Recrystallisation of the residue from diethyl ether/pentane afforded the title compond as a pale brown solid (2.54g).
¹H Nmr (CDCl₃) δ: 1.40 (9H, s), 2.70, (1H, dd, J=5,16Hz), 2.90 (2H, m), 3.16 (1H, m), 3.32 (1H, dd, J=6,16Hz), 3.95 (2H, s), 4.16 (1H, br. s), 4.50 (1H, br. s), 6.75 (1H, d J=8Hz), 6.99-7.30 (12H, m).

### Preparation 6

### (±) trans-1-(2-Benzylbenzyl)-2-(N-methyl-N-tert-butoxycarbonylamino)indane

The title compound was prepared in a similar manner to Preparation 4 from (±)*trans*-1-(2-benzylbenzyl)-2-*tert*-butoxycarbonylaminoindane (750 mg, 1.8mmol), sodium hydride (80% disp. 55mg, 1.8mmol), iodomethane (112 µl, 2 mmol) and N,N-dimethylformamide (7ml). Column chromatography on silica gel eluting with 10% diethyl ether in hexanes afforded the **title compound** as a colourless oil (706mg).
¹H Nmr (CDCl₃) δ : 1.40 (9H, s), 2.51 (3H, br. s), 2.74-3.21 (4H, m), 3.49 (1H, m), 3.98 (2H, s), 4.58+4.88 (1H, br.s +br. s, rotomers), 6.86 (1H, d, J=7Hz), 6.99-7.35 (12H, m).

### Preparation 7

### (±) trans-1-(3,4-Dichlorobenzyl)-2-tert-butoxycarbonylaminoindane

The title compound was prepared in a similar manner to Preparation 5 from (±)N-*tert*-butoxycarbonyl-1,2-iminoindane (2.31g, 10mmol), copper (I) bromide dimethylsulfide complex (411mg, 2 mmol), toluene (70ml) and the Grignard derivative of 3,4-dichlorobenzylbromide (10 mmol) in diethyl ether (10ml). Recrystallisation from diethyl ether/pentane afforded the **title compound** as a pale brown solid (2.54g).
¹H Nmr (CDCl₃) δ: 1.42 (9H, s), 2.70 (1H, dd, J=5, 16Hz), 2.93 (2H, m), 3.23 (2H, m), 4.18 (1H, m), 4.61 (1H, m), 6.89 (1H, d, J=7 Hz), 6.99 (1H, dd, J=1,7 Hz), 7.19 (4H, m), 7.34 (1H, d,J=7Hz).

### Preparation 8

### (±) trans-1-(3,4-Dichlorobenzyl)-2-(N-methyl-N-tert-butoxycarbonylamino)indane

The title compound was prepared in a similar manner to Preparation 4 from (±)*trans*-1-(3,4-dichlorobenzyl)-2-*tert*-butoxycarbonylaminoindane (757 mg, 1.93mmol), sodium hydride (80% disp. 64mg, 2.12mmol), iodomethane (133µl, 2.12 mmol) and N,N-dimethylformamide (7ml). Column chromatography on silica gel eluting with 10% diethyl ether in hexanes afforded the **title compound** as a colourless oil (730mg). ¹H Nmr (CDCl₃) δ: 1.41 (9H, s), 2.61 (3H, br. s), 2.96 (4H, m), 3.50 (1H, q, J=7Hz), 4.58+4.82 (1H, br. s+br. s, rotomers), 6.98-7.40 (7H, m).

### Preparation 9

### (±)trans-1-(4-Benzylbenzyl)-2-tert-butoxycarbonylaminoindane

The title compound was prepared in a similar manner to Preparation 5 from (±) N-*tert*-butoxycarbonyl-1,2-iminoindane (2.31g, 10mmol), copper (I) bromide dimethylsulfide complex (411mg, 2 mmol), toluene (80ml) and the Grignard derivative of 4-benzylbenzylchloride (10 mmol) in diethyl ether (15ml). Column chromatography on silica gel eluting with 20% diethyl ether in hexanes afforded the **title compound** as a white solid (1.36g), m.p.101.5-103.5°C
¹H Nmr (CDCl₃) δ: 1.40 (9H, s), 2.70 (1H, dd, J=5, 16Hz), 2.87 (1H, dd, 7, 15Hz), 2.99 (1H, dd, J= 6, 15Hz), 3.12 (2H, m), 3.95 (2H, s), 4.16 (1H, br. s), 4.58 (1H, br. s), 5.90 (1H, d, J=7 Hz), 7.02-7.33 (12H, m).

### Preparation 10

### (±)trans-1-(4-Benzylbenzyl)-2-(N-methyl-N-tert-butoxycarbonylamino)indane

The title compound was prepared in a similar manner to Preparation 4 from (±) *trans*-1-(4-benzylbenzyl)-2-*tert*-butoxycarbonylaminoindane (685 mg, 1.66mmol), sodium hydride (80% disp. 60mg, 2 mmol), iodomethane (125µl, 2 mmol) and N,N-dimethylformamide (13ml). Column chromatography on silica gel eluting with 10% diethyl ether in hexanes afforded the **title compound** as a pale yellow oil (581mg). ¹H Nmr (CDCl₃) δ: 1.38 (9H, s), 2.52 + 2.62 (3H, br.s + br. s, rotomers), 2.94 (4H, m), 3.50 (1H, q, J=7Hz), 3.95 (2H, s), 4.58 + 4.82 (1H, br.s + br. s, rotomers), 6.96-7.32 (13H, m).

### Preparation 11

### (±) trans-1-(2-Benzylbenzyl)-2-(N-pentyl-N-tert-butoxycarbonylamino)indane

The title compound was prepared in a similar manner to Preparation 4 from (±) *trans*-1-(2-benzylbenzyl)-2-*tert*-butoxycarbonylaminoindane (1g, 2.4mmol), sodium hydride (80% disp. 80mg, 2.6 mmol), 1-bromopentane (277µl, 2.6 mmol) and N,N-dimethylformamide (10ml). Column chromatography on silica gel eluting with 10% diethyl ether in hexanes afforded the **title compound** as a pale yellow oil (651mg). ¹H Nmr (CDCl₃) δ: 0.82 (3H, t, J=6Hz), 0.98-1.50 (15H, m), 2.67 (1H, br. s), 2.80-3.19 (5H, m), 3.64 (1H, q, J=7 Hz), 3.96 (2H, s), 4.17 + 4.65 (1H, br. s + br. s, rotomers), 6.83 (1H, d, J=7 Hz), 6.99-7.32 (12H, m).

### Preparation 12

### (±) trans-1-(4-Benzyloxybenzyl)-2-tert-butoxycarbonylaminoindane

The title compound was prepared in a similar manner to Preparation 5 from (±) N-*tert*-butoxycarbonyl-1,2-iminoindane (2.77g, 12mmol), copper (I) bromide dimethylsulfide complex (411mg, 2 mmol), toluene (80ml) and the unstable Grignard derivative of 4-benzyloxybenzylchloride (10 mmol) in tetrahydrofuran (15ml). Column chromatography on silica gel eluting with 10% diethyl ether in hexanes followed by recrystallisation from diethyl ether/ hexane afforded the **title compound** as a white solid (1.97g), m.p.107-108°C
¹H Nmr (CDCl₃) δ: 1.41 (9H, s), 2.68 (1H, dd, J=5, 16 Hz), 2.84 (1H, dd, J=6, 15 Hz), 2.98 (1H, dd, J=7, 15 Hz), 3.20 (2H, m), 4.16 (1H,m), 4.60 (1H, m), 5.03 (2H, s), 6.83-7.48 (13H, m).

### Preparation 13

### (±) trans-1-(4-Benzyloxybenzyl)-2-(N-methyl-N-tert-butoxycarbonylamino)indane

The title compound was prepared in a similar manner to Preparation 4 from (±) *trans*-1-(4-benzyloxybenzyl)-2-*tert*-butoxycarbonylaminoindane (717 mg, 1.7mmol), sodium hydride (80% disp. 61mg, 2.04mmol), iodomethane (127µl, 2.04mmol) and N,N-dimethylformamide (10ml). Column chromatography on silica gel eluting with 10% diethyl ether in hexanes afforded the **title compound** as a colourless oil (549mg). ¹H Nmr (DMSO-d₆) δ : 1.30 (9H, s), 2.82 (4H, m), 3.31 (3H, s), 3.50 (1H, q, J=7 Hz), 4.50 (1H, m), 5.04 (2H, s), 6.88 (2H, d, J=9 Hz), 7.14 (6H, m), 7.36 (5H, m).

### Preparation 14

### (±) trans-1-(4-Benzyloxybenzyl)-2-(N-ethyl-N-tert-butoxycarbonylamino)indane

The title compound was prepared in a similar manner to Preparation 4 from (±) *trans*-1-(4-benzyloxybenzyl)-2-*tert*-butoxycarbonylaminoindane (620 mg, 1.4mmol), sodium hydride (80% disp. 51mg, 1.7 mmol), bromoethane (127µl, 1.7mmol) and N,N-dimethylformamide (10ml). Column chromatography on silica gel eluting with 10% diethyl ether in hexanes afforded the **title compond** as a colourless oil (357mg). ¹H Nmr (CDCl₃) δ : 1.08 (3H, t, J=6 Hz), 1.45 (9H, s), 3.00 (6H, m), 3.60 (1H, q, J=7 Hz) 4.32 + 4.68 (1H, br. s + br. s, rotomers), 5.07 (2H, s), 6.87-7.52 (13H, m)

### Preparation 15

### (±) E,Z-1-(4-Benzyloxybenzyl)-2-indanone oxime-O-methyl ether

To a solution of lithium bis-(trimethylsilyl)amide (7.44ml of a 1M solution in tetrahydrofuran, 7.44 mmol ) in dry tetrahydrofuran (30ml) at -78°C under nitrogen was added a solution of 2-indanone oxime-O-methyl ether (1g, 6.2 mmol) in tetrahydrofuran (10ml) to afford a green solution. After 10 minutes a solution of 4-benzyloxybenzyl chloride (1.59g, 8.82 mmol) in tetrahydrofuran (10 ml) was added in one portion and the cooling bath removed. When the reaction mixture had reached room temperature, stirring was continued for a further 30 minutes before pouring into a large excess of water and extracting with diethyl ether (3 x 50 ml). The combined organic extracts were dried over sodium sulfate and volatiles removed *in vacuo.* The residue was subjected to column chromatography on silica gel eluting with 10% diethyl ether in hexanes to afford the **title compound** as a yellow oil (1.94g). ¹H Nmr (Major isomer) (CDCl₃) δ : 3.14 (3H, m), 3.46 (1H, d, J=20 Hz), 3.96 (3H, m), 4.41 (1H, t, J=6 Hz), 4.97 (2H, s), 6.70-7.47 (13H, m).

### Preparation 16

### (±)cis-1-(4-Benzyloxybenzyl)-2-tert-butoxycarbonylaminoindane

(±)*cis*-1-(4-Benzyloxybenzyl)-2-aminoindane (250mg, 0.76 mmol) was dissolved in dioxane (10 ml) and cooled to 0°C. Aqueous 3M NaOH (250µl) and di-*tert*-butyldicarbonate (174µl, 0.76 mmol) were added, and the reaction stirred at room temperature for 3 h before pouring into water and extracting with diethyl ether (3 x 40 ml). After drying over sodium sulfate, solvents were removed *in vacuo* and the residue recrystallised from ethanol/hexanes to afford the **title compound** as a white solid (m.p. 146-147°C).
¹H Nmr (CDCl₃) δ : 1.45 (9H, s), 2.72 (2H, m), 2.93 (1H, dd, J=6, 13 Hz), 3.11 (1H, dd, J=6, 15 Hz), 3.52 (1H, m), 4.51 (1H, m), 4.76 (1H, d, J=9 Hz), 5.05 (2H, s), 6.79 (1H, d, J=8 Hz), 6.89 (2H, d, J=8 Hz), 7.05 (2H, d, J= 8 Hz), 7.07-7.498 (8H, m).

### Preparation 17

### 3-(4-Benzyloxyphenyl)propan-1-ol

To a solution of 3-(4-hydroxyphenyl)propan-1-ol (5g, 33mmol) and benzyl bromide (5.1ml, 42.9mmol) in acetone (100 ml) was added potassium carbonate (11.86g, 85.8 mmol). The mixture was then heated at reflux with vigorous stirring for 8 h. On cooling, the mixture was poured into water and extracted with diethyl ether (3 x 50ml). After drying over sodium sulfate, solvents were removed *in vacuo* and the solid residue recrystallised from ethanol/hexanes to afford the **title compound** as a white solid (7.40g)
¹H Nmr (CDCl₃) δ : 1.40 (1H, br. s), 1.89 (2H, m), 2.67 (2H, t, J=8 Hz), 3.68 (2H, t, J=6 Hz), 5.05 (2H, s), 6.91 (2H, d, J=9 Hz), 7.12 (2H, d, J=9 Hz), 7.42 (5H, m).

### Preparation 18

### 1-(4-Benzyloxyphenyl)-3-bromopropane

To a solution of 3-(4-benzyloxyphenyl)propan-1-ol (2.75g, 11.36mmol) and triphenylphosphine (3.27g, 12.5mmol) in N,N-dimethylformamide (30ml) was added bromine dropwise until a faint orange colour persisted. The reaction mixture was allowed to stir at room temperature for 1 hour then poured into water and extracted with pentane. The combined organic phases were dried over sodium sulfate and concentrated *in vacuo.* The residue was subjected to column chromatography on silica gel eluting with 5% diethyl ether in hexanes to afford the **title compound** as a colourless oil which solidified on standing. (2.49g).
¹H Nmr (CDCl₃) δ : 2.15 (2H, m), 2.73 (2H, t, J=8 Hz), 3.38 (2H, t, J=6 Hz), 5.05 (2H, s), 6.91 (2H, d, J=9 Hz), 7.12 (2H, d, J=9 Hz), 7.42 (5H, m).

### Preparation 19

### (±) 1-[3-(4-Benzyloxyphenyl)propyl]-2-indanone oxime-O-methyl ether

The title compound was prepared in a similar manner to Preparation 15 from 2-indanone oxime-O-methyl ether (960mg, 6 mmol), lithium bis(trimethylsilylamide) (1M in tetrahydrofuran, 7.2 ml, 7.2 mmol), tetrahydrofuran (60 ml) and 1-(4-benzyloxyphenyl)-3-bromopropane (2g, 6.6 mmol). Column chromatography on silica gel eluting with 5% diethyl ether in hexanes afforded the **title compound** as a pale yellow oil (821mg).
¹H Nmr (CDCl₃) δ : 1.48 (2H, m), 1.85 (1H, m), 2.02 1H, m), 2.50 (2H, t, J=8 Hz), 3.62 1H, d, J=22 Hz), 3.76 (1H, d, J=22 Hz), 3.86 (3H, s, ), 4.20 1H, t, J=6 Hz), 5.03 (2H , s), 6.85 (2H, d, J=10 Hz), 7.03 (2H, d, J= 10 Hz), 7.19 (4H, m), 7.37 (5H, m).

### Preparation 20

### (±)cis-1-[3-(4-Benzyloxyphenyl)propyl]-2-ethoxycarbonylaminoindane

To a solution of (±) *cis*-1-[3-(4-benzyloxyphenyl)propyl]-2-aminoindane (400mg, 1.1mmol) and triethylamine (696µl, Smmol) in diethyl ether (30ml) was added ethyl chloroformate (143µl, 1.5 mmol) dropwise. After stirring at room temperature for 30 minutes the reaction mixture was poured into water and extracted with diethyl ether. The combined organic extracts were dried over sodium sulfate and concentrated in *vacuo* to afford the **title compound** as an off-white solid (451mg).
¹H Nmr (CDCl₃) δ : 1.22 (3H, t, J=7 Hz), 1.75 (4H, m), 2.62 (2H, t, J=7 Hz), 2.80 (1H, dd, J=5, 16Hz), 3.14 (2H, m), 4.10 (2H, q, J=7 Hz), 4.60 (1H, m), 4.73 (1H, m), 5.05 (2H, s), 6.90 (2H, d, J=9 Hz), 7.05-7.50 (11H, m).

### Preparation 21

### (±) E,Z-1-(4-Methoxybenzyl)-2-indanone oxime-O-methyl ether

The title compound was prepared in a similar manner to Preparation 15 from 2-indanone oxime-O-methyl ether (3.02g, 18.7 mmol), lithium bis(trimethylsilylamide) (1M solution in tetrahydrofuran, 18.6 ml, 18.6 mmol), tetrahydrofuran (150 ml) and 4-methoxybenzyl chloride (3.22g, 20.6 mmol). Column chromatography on silica gel eluting with 20% diethyl ether in hexanes afforded the **title compound** as a pale yellow oil (4.50g).
¹H Nmr (major isomer)(CDCl₃) δ: 3.11-3.19 (3H, m), 3.45 (1H, d, J=20Hz), 3.74 (3H, s), 3.98 (3H, s), 4.42 (1H, t, J=5Hz), 6.67 (1H, d, J=9Hz), 6.79 (1H, d, J=9Hz), 6.97-7.00 (2H, m), 7.11-7.18 (4H, m).

### Preparation 22

### (±)-cis-1-(4-Methoxybenzyl)-2-ethoxycarbonylaminoindane

The title compound was prepared in a similar manner to Preparation 20 from (±)-cis-1-(4-methoxybenzyl)-2-aminoindane (540mg, 2.lmmol), triethylamine (1.4ml, 10mmol) in dry diethyl ether (60ml) and ethyl chloroformate (0.22ml, 2.3mmol). The crude product was subjected to column chromatography on silica gel eluting with 20% diethyl ether in 60-80° petrol to afford the **title compound** (0.43g).
¹H Nmr (CDCl₃) δ: 1.25 (3H, t, J=7Hz), 2.71-2.82 (2H, m), 2.94 (1H, dd, J=7,15Hz), 3.14 (1H, dd, J=7, 15Hz), 3.48-3.60 (1H, m), 3.80 (3H, s), 4.13 (2H, q, J=7Hz), 4.52-4.62 (1H, m), 4.89 (1H, d, J=9Hz), 6.82 (3H, d, J=9Hz), 7.04-7.23 (5H, m).

### Preparation 23

### (±) 1-Hydroxymethyl-2-indanone oxime-O-methyl ether

To a solution of lithium bis(trimethylsilylamide) (1M in tetrahydrofuran, 55 ml, 55 mmol) in dry tetrahydrofuran (200 ml) at -60°C under argon was added a solution of 2-indanone-oxime-O-methyl ether (8.05g, 50 mmol) in tetrahydrofuran (50ml). After 10 minutes, the temperature of the cold bath was adjusted to -15°C. Gaseous formaldehyde (from heating dried paraformaldehyde at 150°C) was then bubbled through the solution on a rapid stream of argon. After 30 minutes, the reaction mixture was poured into water (500ml) and extracted three times with diethyl ether. The combined organic extracts were dried over sodium sulfate, concentrated *in vacuo* and the residue subjected to column chromatography on silica gel eluting with 40% ether in petrol to afford the **title compound** (5.1g) as a brown solid.
¹H Nmr (CDCl₃) δ : 2.70 (1H, dd, J=5,8Hz), 3.55-4.10 (3H, m), 3.93 (3H, s), 4.36 (1H, m), 7.26 (4H, m).

### Preparation 24

### (±) cis-1-Hydroxymethyl-2-aminoindane

The title compound was prepared in a similar manner to Example 13 from (±) 1-hydroxymethyl-2-indanone oxime-O-methyl ether (4.62g, 24mmol), lithium aluminium hydride (2.74g, 72mmol) and diethyl ether (500ml). The crude material (3.76g) was used without purification.
¹H Nmr (CDCl₃) δ : 2.46 (3H, br. s), 2.65 (1H, dd, J=7,15Hz), 3.07 (1H, m), 3.20 (1H, dd, J=7,15Hz), 3.59 (1H, q, 7Hz), 3.82 (1H, dd, J=9,10Hz), 4.15 (1H, dd, J=5,10Hz), 7.19 (4H, m).

### Preparation 25

### (±) cis-1-[(4-benzyl)phenoxymethyl)]-2-tert-butoxycarbonylaminoindane

The title compound was prepared in a similar manner to Preparation 16 from (±) *cis*-1-[(4-benzyl)phenoxymethyl)]-2-aminoindane (lg, 3mmol), aqueous 3M NaOH (1ml, 3mmol), di-tert-butyl dicarbonate (688µl, 4.4mmol) and 1,4-dioxane (50ml). Recrystallisation of the crude product from ethanol-60/80 petrol afforded the **title compound** as a white solid (1.17g), m.p. 121.5-122.5°C.
¹H Nmr (CDCl₃) δ : 1.44 (9H, s), 2.80 (1H, dd, J=5,15Hz), 3.40 (2H, m), 3.91 (2H, s), 4.17 (2H, m), 4.30 (1H, m), 4.88 (1H, br. s), 6.84 (2H, d, J=8Hz), 7.09 (2H, d, J=8Hz), 7.13-7.40 (9H, m).

### Preparation 26

### (±) E,Z-1-(4-Phenoxybenzyl)-2-indanone oxime-O-methyl ether

The title compound was prepared in a similar manner to Preparation 15 from 2-indanone oxime-O-methyl ether (3.04g, 19 mmol), lithium bis(trimethylsilylamide) (1M in tetrahydrofuran, 25 ml, 25 mmol), tetrahydrofuran (170 ml) and 4-phenoxybenzyl chloride (5.12g, 23 mmol). Column chromatography on silica gel eluting with 10% diethyl ether in hexanes afforded the **title compound** as a pale yellow oil (6.30g).
¹H Nmr (Major isomer)(CDCl₃) δ: 3.15-3.23 (3H, m), 3.48 (1H, d, J=20Hz), 3.97 (3H, s), 4.45 (1H, t, J=5Hz), 6.77-7.32 (13H, m).

### Preparation 27

### (±) cis-1-[(4-Phenoxy)benzyl]-2-tert-butoxycarbonylaminoindane

The title compound was prepared in a similar manner to Preparation 16 from (±) *cis*-1-[(4-phenoxy)benzyl]-2-aminoindane (648mg, 2.06mmol), aqueous 3M NaOH (687µl), di-tert-butyldicarbonate (474µl, 2.06mmol) and 1,4-dioxane (40ml). Recrystallisation of the crude product from ethanol-60/80 petrol afforded the **title compound** as a white solid (483mg).
¹H Nmr (CDCl₃) δ: 1.45 (9H, s), 2.70-2.81 (2H, m), 2.96 (1H, dd, J=7,15Hz), 3.12 (1H, dd, J=7,15Hz), 3.52-3.60 (1H, m), 4.52 (1H, m), 4.76 (1H, d, J=9Hz), 6.83 (1H, d, J=7Hz), 6.91-7.36 (12H, m).

### Preparation 28

### (±)E,Z-1-(3,4-Dichlorobenzyl)-2-indanone oxime-O-methyl ether

The title compound was prepared in a similar manner to Preparation 15 from 2-indanone oxime-O-methyl ether (5.05g, 31 mmol), lithium bis(trimethylsilylamide) (1M in tetrahydrofuran, 41 ml, 41 mmol), tetrahydrofuran (250 ml) and 3,4-dichlorobenzyl chloride (7.34g, 38 mmol). Column chromatography on silica gel eluting with 10% diethyl ether in hexanes afforded the **title compound** as a pale yellow oil (8.04g).
¹H Nmr (Major isomer)(CDCl₃) δ: 3.14-3.18 (2H, m), 3.51 (2H, d, J=20Hz), 3.97 (3H, s), 4.43 (1H, t, J=5Hz), 6.60-7.23 (7H, m).

### Preparation 29

### (±)cis-1-(3,4-Dichlorobenzyl)-2-tert-butoxycarbonylaminoindane

The title compound was prepared in a similar manner to Preparation 16 from (±) *cis*-1-(3,4-dichlorobenzyl)-2-aminoindane (1.29g, 4.4mmol), aqueous 3M NaOH (1.48ml), di-tert-butyldicarbonate (1.02ml, 4.4mmol) and 1,4-dioxane (100ml)). Recrystallisation of the crude product from ethanol/petrol afforded the **title compound** as a white solid (1.65g).
¹H Nmr (CDCl₃) δ : 1.54 (9H, s), 2.68-2.81 (2H, m), 2.92 (1H, dd, J=7,15Hz), 3.14 (1H, dd, J=7,15Hz), 3.44-3.59 (1H, m), 4.47-4.53 (1H, m), 4.73 (1H, d, J=9Hz), 6.81 (1H, d, J=7Hz), 6.99 (1H, d, J=9Hz), 7.08-7.26 (4H, m), 7.45 (1H, d, J=9Hz).

### Preparation 30

### (±) cis-1-(3,4-Dichlorobenzyl)-2-(N-methyl-N-tert-butoxycarbonylamino)indane

The title compound was prepared in a similar manner to Preparation 4 from (±) *cis*-1-(3,4-dichlorobenzyl)-2-*tert*-butoxycarbonylaminoindane (1.65g, 4.2mmol), sodium hydride (80% disp. in oil, 130mg, 4.3mmol), iodomethane (280µl, 4.5mmol) and N,N-dimethylformamide (16ml). Column chromatography on silica gel eluting with 10% diethyl ether in hexanes afforded the **title compound** as a colourless oil (1.016g).
¹H Nmr (CDCl₃) δ : 1.45 (9H, s), 2.65 (3H, s), 2.70-2.91 (2H, m), 3.04 (1H, dd, J=6,15Hz), 3.18 (1H, dd, J=7,15Hz), 3.64 (1H, m), 4.98 (1H, br.s), 6.78 (1H, d, J=7Hz), 7.02-7.29 (5H, m), 7.37 (1H, d, J=9Hz).

### Preparation 31

### (±)E,Z-1-(4-Trifluoromethylbenzyl)-2-indanone oxime-O-methyl ether

The title compound was prepared in a similar manner to Preparation 15 from 2-indanone oxime-O-methyl ether (5.03g, 31 mmol), lithium bis(trimethylsilylamide) (1M solution in tetrahydrofuran, 41 ml, 41 mmol), tetrahydrofuran (250 ml) and α'-bromo-α,α,α-trifluoro-p-xylene (9.32g, 39 mmol). Column chromatography on silica gel eluting with 10% diethyl ether in hexanes afford the **title compound** as a pale yellow oil (6.24g).
¹H Nmr (Major isomer)(CDCl₃) δ : 3.05-3.40 (3H, m), 3.48 (1H, d, J=20Hz), 3.96 (3H, s), 4.48 (1H, t, J=5Hz), 6.97-7.00 (2H, m), 7.14-7.24 (4H, m), 7.38-7.46 (2H, m).

### Preparation 32

### (±) cis-1-(4-Benzyloxybenzyl)-2-(N-methyl-N-tert-butoxycarbonylamino)indane

The title compound was prepared in a similar manner to Preparation 4 from (±) *cis*-1-(4-benzyloxybenzyl)-2-*tert*-butoxycarbonylaminoindane (500mg, 1.1mmol), sodium hydride (80% disp. in oil, 39mg, 1.3mmol), iodomethane (81µl, 1.3mmol) and N,N-dimethylformamide (10ml). Column chromatography on silica gel eluting with 10% diethyl ether in hexanes afforded the **title compound** as a colourless oil (370mg).
1H Nmr (CDCl₃) δ : 1.47 (9H, s), 2.68 (4H, m), 2.90 (1H, dd, J=6,14Hz), 3.12 (2H, m), 3.63 (1H, br. s), 4.97 (1H, br. s), 5.08 (2H, s), 6.75 (1H, d, J=7Hz), 6.92 (2H, d, J=8Hz), 7.01-7.52 (10H, m).

### Preparation 33

### (±) E,Z-1-(4-Hydroxybenzyl)-2-indanone oxime-O-methyl ether

To a suspension of 10% palladium on charcoal (1g) in methanol (150ml) under argon was added (±)E,Z-1-(4-benzyloxybenzyl)-2-indanone oxime-O-methyl ether (4g, 11.2mmol) and ammonium formate (4g). The mixture was then heated at reflux for 1 hour. On cooling the catalyst was filtered off and the solvent removed *in vacuo.* The residue was then partitioned between diethyl ether and water. The ether extracts were dried over MgSO₄ and the volatiles were removed *in vacuo.* The residue was then subjected to column chromatography on silica gel eluting with 40% diethyl ether in 60-80 petrol to afford the **title compound** as white solid (2.1g).
¹H Nmr (CDCl₃) δ: 3.08 (2H, m), 3.25 (1H, dd, J=7,14Hz), 3.46 (1H, d, J=20Hz), 4.00 (3H, s), 4.42 (1H, m), 5.44 (1H, m), 6.68 (3H, m), 6.90 (1H, t, J=8Hz), 7.13 (4H, m).

### Preparation 34

### (±) E,Z-1-[(4-Cyclopropylmethoxy)benzyl]-2-indanone oxime-O-methyl ether

To a solution of (±) E,Z-1-(4-hydroxybenzyl)-2-indanone oxime-O-methyl ether (2g, 7.5mmol) in dry N,N-dimethylformamide (30ml) under argon was added potassium carbonate (2.07g, 15mmol) and cyclopropylmethyl bromide (1.45ml, 15mmol). The mixture was stirred at room temperature for 3 h then at 60°C for 1.5 h. The reaction mixture was then partitioned between diethyl ether and water. The organic phase was dried over MgSO₄, concentrated *in vacuo,* and the residue subjected to column chromatography on silica gel eluting with 25% diethyl ether in 60-80 petrol to afford the **title compound** as a brown oil (1.34g).
¹H Nmr (CDCl₃) (one isomer) δ : 0.35 (2H, m), 0.61 (2H, m), 1.25 (1H, m), 2.91 (1H, dd, J=7,15Hz), 3.24 (2H, m), 3.50 (1H, d, J=20Hz), 3.72 (2H, t, J=7Hz), 4.00 (3H, s), 4.14 (1H, m), 6.73 (3H, m), 6.93 (2H, m), 7.14 (3H, m).

### Preparation 35

### (±) E,Z-1-(4-n-Butoxybenzyl)-2-indanone oxime-O-methyl ether

To a solution of (±) E,Z-1-(4-hydroxybenzyl)-2-indanone oxime-O-methyl ether (2g, 7.5mmol) in dry N,N-dimethylformamide (30ml) under argon was added potassium carbonate (2.07g, 15mmol) and 1-bromobutane (1.61ml, 15mmol). The mixture was stirred at 50°C overnight. The reaction mixture was then partitioned between diethyl ether and water. The organic phase was dried over MgSO₄, concentrated *in vacuo,* and the residue subjected to column chromatography on silica gel eluting with 25% diethyl ether in 60-80 petrol to afford the **title compound** as a brown oil (1.55g).
¹H Nmr (CDCl₃) (one isomer) δ:0.95 (3H, m), 1.48 (2H, m), 1.76 (2H, m), 2.91 (1H, dd, J=7,14Hz), 3.21 (2H, m), 3.70 (1H, d, J=22Hz), 3.91 (5H, m), 4.14 (1H, m), 6.72 (3H, m), 6.96 (2H, m), 7.15 (3H, m).

### Preparation 36

### (±) E,Z-1-(3,4-Dimethoxybenzyl)-2-indanone oxime-O-methyl ether

The title compound was prepared in a similar manner to Preparation 15 from 2-indanone oxime-O-methyl ether (3.0g, 19 mmol), lithium bis-(trimethylsilylamide) (1M solution in tetrahydrofuran, 21 ml, 21 mmol), tetrahydrofuran (150 ml) and 3,4-dimethoxybenzyl chloride (5.04g, 27 mmol). Column chromatography on silica gel eluting with 50% diethyl ether in hexanes afforded the **title compound** as a pale yellow oil (2.59g).
¹H Nmr (major isomer)(CDCl₃) δ: 3.05 (1H, d, J=20Hz), 3.12 (1H, m), 3.25 (1H, dd, J=7,15Hz), 3.44 (1H, d, J=20Hz), 3.59 (3H, s), 3.84 (3H, s), 3.98 (3H, s), 4.45 (1H, m), 6.20 (1H, d, J=1Hz), 6.51 (1H, dd, J=1,7Hz), 6.69 (1H, d, J=7Hz), 7.15 (4H, m).

### Preparation 37

### (±) E,Z-1-[(5-Chloro-2-thienyl)methyl]-2-indanone oxime-O-methyl ether

The title compound was prepared in a similar manner to Preparation 15 from 2-indanone oxime-O-methyl ether (4.39g, 27mmol), lithium bis-(trimethylsilylamide) (1M solution in tetrahydrofuran, 32.0ml, 32 mmol), tetrahydrofuran (100 ml) and 2-chloro-5-(chloromethyl)thiophene (5.0g, 30 mmol). After stirring at room temperature for 75min the reaction was quenched with excess glacial acetic acid and concentrated *in vacuo.* The residue was partitioned between water and diethyl ether. The pH of the aqueous layer was adjusted to 8 with potassium carbonate and it was extracted with diethyl ether (3x 200ml). The combined organic layers were dried over sodium sulfate and concentrated *in vacuo* to give an oil. Purification by column chromatography on silica gel eluting with 10% diethyl ether in 40-60 petrol afforded the **title compound** as a pale yellow oil (4.50g).

### Preparation 38

### (±) cis-1-[(5-Chloro-2-thienyl)methyl]-2-tert-butoxycarbonylaminoindane

The title compound was prepared in a similar manner to Preparation 16 from (±) *cis*-2-amino-1-[(5-chloro-2-thienyl)methyl]indane (1g, 3.8mmol), aqueous 3M NaOH (1.3ml, 3.9mmol), di-*tert*-butyldicarbonate (0.83g, 3.8mmol) and 1,4-dioxane (50ml). Purification of the crude product by chromatography on silica gel eluting with 10-20% diethyl ether in 40-60 petrol afforded the **title compound** (1.2g).
¹H Nmr (CDCl₃) δ : 1.51 (9H, s), 2.71 (1H, dd, J=6,15Hz), 2.90-3.28 (3H, m), 3.55 (1H, m), 4.58 (1H, br m), 4.71 (1H, br m), 6.52 (1H, br d, J=3Hz), 6.70 (1H, d, J=3Hz), 7.02 (1H, br d, J=7Hz), 7.10-7.30 (3H, m).

### Preparation 39

### (±) cis-1-[(5-Chloro-2-thienyl)methyl]-2-(N-methyl-N-tert-butoxycarbonylamino)indane

A solution of (±) *cis*-1-[(5-chloro-2-thienyl)methyl]-2-*tert*-butoxycarbonylaminoindane (1.2g, 3.3mmol) in N,N-dimethylformamide (25ml) was treated with sodium hydride (124mg of an 80% dispersion in oil, 4.125mmol) and stirred at room temperature for 1.75h. Iodomethane (0.25ml, 3.96mmol) was added and the reaction was stirred at room temperature. After 18h further portions of sodium hydride (50mg of an 80% dispersion in oil, 1.65mmol) and iodomethane (0.1ml, 1.65mmol) was added and the reaction was stirred for a further 24h. The reaction was poured into ice-water, treated with brine and extracted into chloroform (3x100ml). The combined extracts were dried over sodium sulfate and concentrated *in vacuo* using co-distillation with toluene to remove residual traces of N,N-dimethylformamide. Chromatography on silica gel eluting with 10-20% diethyl ether in 40-60 petrol afforded the **title compound** as a pale yellow oil (0.72g).
¹H Nmr (CDCl₃) δ: 1.47 (9H, s), 2.60 (3H, s), 2.85-3.10 (3H, m), 3.20 (1H, dd, J= 7,15Hz), 3.62 (1H, m), 5.02 (1H, br m), 6.58 (1H, br d, J=3Hz), 6.73 (1H, d, J=3Hz), 6.95 (1H, d, J=7Hz), 7.06-7.30 (3H, m).

### Preparation 40

### (±) cis-1-(3,4-Dichlorobenzyl)-2-(N-cyclopropylmethyl-N-tert-butoxycarbonylamino) indane

The title compound was prepared in a similar manner to Preparation 4 from (±) *cis*-1-(3,4-dichlorobenzyl)-2-*tert*-butoxycatbonylaminoindane (1.49g, 3.8mmol), sodium hydride (80% disp. in oil, 120mg, 4.0mmol), bromomethylcyclopropane (407µl, 4.2mmol) and N,N-dimethylformamide (17ml). Column chromatography on silica gel eluting with 10% diethyl ether in hexanes afforded the **title compound** as a colourless oil (686mg).
¹H Nmr (CDCl₃) δ : 0.21 (2H, t, J=7Hz), 0.46 (2H, dd, J=1,9Hz), 0.85 (1H, m), 1.46 (9H, s), 2.54 (1H, dd, J=10,13Hz), 2.84-3.00 (2H, m), 3.14-3.25 (3H, m), 3.65 (1H, m), 4.80 (1H, m), 6.59 (1H, d, J=7Hz), 6.94 (1H, d, J=9Hz), 7.04 (1H, t, J=7Hz), 7.13-7.25 (3H, m), 7.34 (1H, d, J=9Hz)

### Preparation 41

### 4-(4-Fluorophenoxy)benzaldehyde

A solution of 4-fluorobenzaldehyde (1.24g, 0.lmol), 4-fluorophenol (1.12g, 0.1mol) in dry dimethylsulfoxide (5ml) was treated with potassium carbonate (1.38g, 0.1mol) and 18-crown-6 (5mg) and heated overnight under an argon atmosphere in an oil bath at 100°C. The reaction was poured into water (100ml) and extracted into diethyl ether (3x50ml). The combined ether layers were washed with water (50ml) then brine (50ml) and dried over sodium sulfate. Concentration *in vacuo* afforded the **title compound** as a light brown solid (2.0g) which was used in the next stage without further purification.
¹H Nmr (CDCl₃) δ : 7.05 (6H, m), 7.85 (2H, d, J=8Hz), 9.92 (1H, s).

### Preparation 42

### 4-(4-Fluorophenoxy)benzyl alcohol

To a solution of 4-(4-fluorophenoxy)benzaldehyde (2.0g, 9.25mmol) in methanol (75ml) was added portionwise sodium borohydride (2.0g, 53.65mmol) with ice cooling. The mixture was stirred at room temperature for lh and then concentrated *in vacuo.* The residue was partitioned between water (100ml) and diethyl ether (100ml). The aqueous layer was extracted with diethyl ether (2x100ml) and the combined organic extracts were washed with brine (100ml) and dried over sodium sulfate. Concentration *in vacuo* afforded the **title compound** as a white solid (2.0g) which was used in the next stage without further purification.
¹H Nmr (CDCl₃) δ : 1.78 (1H,t, J=6Hz), 4.63 (2H, d, J=6Hz), 6.98 (6H, m), 7.32 (2H, d, J=7Hz).

### Preparation 43

### 4-(4-Fluorophenoxy)benzyl chloride

To a solution of thionyl chloride (2.0ml, 27.5mmol) in absolute chloroform (15ml) was added a solution of 4-(4-fluorophenoxy)benzyl alcohol (2.0g, 9.17mmol) in chloroform (35ml). The mixture was heated under reflux for 0.5h and then concentrated *in vacuo.* The residue was dissolved in diethyl ether (50ml) and washed with saturated aqueous sodium hydrogen carbonate (50ml) followed by brine (50ml). The organic layer was dried over sodium sulfate and concentrated *in vacuo.* Distillation of the residual oil in a Kugelrohr apparatus at 190°C /0.3mmHg gave the **title compound** as a colourless oil (1.96g) which crystallised on cooling.
¹H Nmr (CDCl₃) δ : 4.58 (2H, s), 6.98 (6H, m), 7.32 (2H, d, J=7Hz).

### Preparation 44

### (±) E,Z-1-[4-(4-fluorophenoxy)benzyl]-2-indanone oxime-O-methyl ether

The title compound was prepared in a similar manner to Preparation 15 from 2-indanone oxime-O-methyl ether (1.2g, 7.5mmol), lithium bis-(trimethylsilylamide) (1M solution in tetrahydrofuran, 9.0ml, 9.0 mmol), tetrahydrofuran (60 ml) and 4-(4-fluorophenoxy)benzyl chloride (1.95g, 8.25mmol). After stirring at room temperature for 2h the reaction was worked up as described in Preparation 15. Purification by chromatography on silica gel eluting with 10% diethyl ether in 40-60 petrol afforded the **title compound** as a pale yellow oil (1.79g).
¹H Nmr (CDCl₃) (major isomer) δ: 3.18 (3H, m), 3.48 (1H, d, J=20Hz), 3.98 (3H, s), 4.43 (1H, t, J=6Hz), 6.68-7.22 (12H, m).

### Preparation 45

### (±) cis-1-[4-(4-Fluorophenoxy)benzyl]-2-tert-butoxycarbonylaminoindane

The title compound was prepared in a similar manner to Preparation 16 from (±)*cis*-1-[4-(4-fluorophenoxy)benzyl]-2-aminoindane (0.19g, 0.57 mmol), aqueous 3M NaOH (0.19ml, 0.57mmol), di-tert-butyldicarbonate (0.12g, 0.57mmol) and 1,4-dioxane (20ml). After work up the **title compound** was obtained as an oil (0.26g) which was dried by azeotropic distillation with toluene prior to the next stage.
¹H Nmr (CDCl₃) δ : 1.45 (9H, s), 2.75 (2H, m), 2.95 (1H, dd, J=7,13Hz), 3.12 (1H,dd, J=7, 15Hz), 3.55 (1H, m), 4.50 (1H, m), 4.75 (1H, br. d, J=8Hz), 6.80-7.25 (12H, m).

### Preparation 46

### (±) cis-1-[4-(4-Fluorophenoxy)benzyl]-2-(N-methyl-N-tert-butoxycarbonylamino)indane

A solution of (±) *cis-*1-[4(4-fluorophenoxy)benzyl]-2-*tert*-butoxycarbonylaminoindane (0.25g, 0.57mmol) in dry N,N-dimethylformamide (8ml) was treated with sodium hydride (21mg of an 80% dispersion in oil, 0.71mmol) and stirred at room temperature for 2h. Iodomethane (0.044ml, 0.71mmol) was added and the reaction was stirred at room temperature for 48h. During this period further portions of sodium hydride (40mg) and iodomethane (0.09ml) were added. The reaction was concentrated *in vacuo* using azeotropic distillation with toluene. The residue was partitioned between water (30ml) and diethyl ether (30ml). The ether layer was washed with brine (20ml) and dried over sodium sulfate. After concentration *in vacuo* the residue was purified by chromatography on silica gel eluting with 10% diethyl ether in 40-60 petrol to give the **title compound** as a pale yellow oil (0.16g).
¹H Nmr (CDCl₃) δ: 1.45(9H, s), 2.65 (3H, br. s), 2.68-3.25 (4H, m), 3.65 (1H, m), 4.98 (1H, br. m), 6.70-7.25 (12H, m).

### Preparation 47

### (±) E,Z-1-Benzyl-2-indanone oxime-O-methyl ether

The title compound was prepared in a similar manner to Preparation 15 from 2-indanone oxime-O-methyl ether (3.05g, 19mmol), lithium bis(trimethylsilyl)amide (25ml of a 1M solution in tetrahydrofuran, 25mmol) and benzyl bromide (2.74ml, 23mmol) in tetrahydrofuran (150ml). After stirring at room temperature for 2 h the reaction was worked up as described in Preparation 15. Purification by column chromatography on silica gel eluting with 10% diethyl ether in hexanes afforded the **title compound** as a yellow oil (3.21g).
¹H Nmr (CDCl₃) (major isomer) δ : 3.11-3.20 (3H, m), 3.47 (1H, d, J=20Hz), 3.97 (3H, s), 4.45 (1H, t , J=7Hz), 6.87-6.95 (4H, m), 7.07-7.18 (5H, m).

### Preparation 48

### (±)cis-1-(4-Trifluoromethylbenzyl)-2-tert-butoxycarbonylaminoindane

The title compound was prepared in a similar manner to Preparation 16 from (±)*cis*-1-(4-trifluoromethylbenzyl)-2-aminoindane (875mg, 3.0mmol), aqueous 3M NaOH (1.0ml, 3.0mmol), di-tert-butyldicarbonate (0.69ml, 3.0mmol) and 1,4-dioxane (60ml). The crude product was subjected to column chromatography on silica gel eluting with 15% diethyl ether in hexanes to afford the **title compound** as a white solid (918mg).
¹H Nmr (CDCl₃) δ : 1.46 (9H, s), 2.80 (2H, m), 2.99-3.18 (2H, m), 3.60 (1H, m), 4.51 (1H, t, J=7Hz), 4.76 (1H, d, J=7Hz), 6.77 (1H, d, J=7Hz), 7.05-7.28 (5H, m), 7.54 (2H, d, J=7Hz).

### Example 1

### (±)trans-1-Benzyl-2-methylaminoindane Hydrochloride (El)

To a solution of LiAlH₄ (380mg, 10mmol) in dry tetrahydrofuran (15 ml) under nitrogen was added dropwise a solution of (±)*trans*-1-benzyl-2-tert-butoxycarbonylamino indane (362mg, 1.1mmol) in dry tetrahydrofuran (10ml). The reaction was heated at reflux for 4 h then cooled with an ice/water bath and quenched with the minimum of water. The reaction was filtered and dried over Na₂SO₄. Solvents were removed *in vacuo* and the residue subjected to column chromatography on silica gel eluting with 5% ethanol in chloroform to afford a colourless oil (220mg) which was converted to the HCl salt and crystallised to afford the **title compound** as a white solid. m.p. 165-166°C (from acetone/diethyl ether).
¹H Nmr (DMSO-d₆) δ: 2.50 (3H, s), 2.96 (1H, dd, J=7,14Hz), 3.09-3.39 (3H, m), 3.78 (1H, m), 3.91 (1H, m), 7.04 (1H, d, J=8Hz), 7.18-7.46 (8H, m), 9.47 (2H, br. s).

### Example 2 (±)trans-1-Benzyl-2-dimethylaminoindane Hydrochloride (E2)

The title compound was prepared in a similar manner to Example 1 from LiAlH₄ (1.02g, 27mmol), (±)*trans*-1-benzyl-2-(N-methyl-N-*tert*-butoxycarbonylamino)indane (911 mg, 2.7mmol) and tetrahydrofuran (55ml). After a reaction time of 5 h the reaction was worked up as previously described and subjected to column chromatography on silica gel eluting with 5% ethanol in chloroform to afford a colourless oil ( 495mg) which was converted to the HCI salt and crystallised to afford the **title compound** as a white solid. m.p. 194-196°C (from acetone-diethyl ether).
¹H Nmr (DMSO-d₆) δ : 2.45 (3H, br. s), 2.55 (3H, br. s), 2.96 (1H, dd, J=7,14Hz), 3.13 (1H, dd, J=7,14Hz), 3.33 (2H, d, J=6Hz), 3.95 (2H, m), 7.00 (1H, d, J=7Hz), 7.16-7.47 (8H, m), 11.39 (1H, br. s).

### Example 3

### (±)trans-1-(2-Benzylbenzyl)-2-methylaminoindane Hydrochloride (E3)

The title compound was prepared in a similar manner to Example 1 from LiAlH₄ (912mg, 24mmol), (±)*trans*-1-(2-benzylbenzyl)-2-*tert*-butoxycarbonylaminoindane (1g, 2.4mmol) and tetrahydrofuran (50ml). After a reaction time of 2 h the reaction was worked up as previously described and subjected to column chromatography on silica gel eluting with 5% ethanol in chloroform to afford a colourless oil (741mg) which was converted to the HCI salt and crystallised to afford the **title compound** as a white solid. m.p. 128-131°C (from acetone-diethyl ether).
¹H Nmr (DMSO-d₆) δ : 2.50 (3H, s), 2.94 (1H, dd, J=7,14Hz), 3.16 (1H, dd, J=7,14Hz), 3.23 (1H, d, J=18Hz), 3.58 (1H, m), 3.77 (2H, m), 4.02 (2H, s), 6.83 (1H, d, J=7Hz), 7.17- 7.50 (12H, m), 9.37 (2H, br. s).

### Example 4

### (±)trans-1-(2-Benzylbenzyl)-2-dimethylaminoindane Hydrochloride (E4)

The title compound was prepared in a similar manner to Example 1 from LiAlH₄ (494mg, 13mmol), (±)*trans*-1-(2-benzylbenzyl)-2-(N-methyl-N-*tert*-butoxycarbonylamino)indane (550mg, 1.3mmol) and tetrahydrofuran (50ml). After a reaction time of 3 h the reaction was worked up as previously described and subjected to column chromatography on silica gel eluting with 5% ethanol in chloroform to afford a colourless oil (404mg) which was converted to the HCl salt and crystallised to afford the title compound as a white solid. m.p. 166-167°C (from acetone-diethyl ether).
¹H Nmr (DMSO-d₆) δ: 2.35+2.50 (6H, br. s+ br. s, protomers), 2.90 (1H, dd, J=7,14Hz), 3.05 (1H, dd, J=7,14Hz), 3.48 (2H, m), 3.88 (2H, m), 3.99 (2H, s), 6.76 (1H, d, J=7Hz), 7.05-7.48 (12H, m), 11.40 (1H, br. s).

The two enantiomers were separated by chiral HPLC on a Chiralcel OJ column (250 x 10 mm) eluting with 5% ethanol in hexanes.

### Example 5

### (±)trans-1-(3,4-Dichlorobenzyl)-2-methylaminoindane Hydrochloride (E5)

### Method A

The title compound was prepared in a similar manner to Example 1 from LiAlH₄ (684mg, 18mmol), (±)*trans*-1-(3,4-dichlorobenzyl)-2-*tert*-butoxycarbonylaminoindane (700mg, 1.8mmol) and tetrahydrofuran (50ml). After a reaction time of 2.5 h the reaction was worked up as previously described and subjected to column chromatography on silica gel eluting with 5% ethanol in chloroform to afford a colourless oil (455mg) which was converted to the HCl salt and crystallised to afford the title compound *(c.* 90% pure by nmr) as a white solid. m.p. 150-153°C (from acetone-diethyl ether).
¹H Nmr (CDCl₃) 2.42 (3H, s), 2.90 (1H, dd, J=8,14Hz), 3.23 (3H, m), 3.60 (1H, m), 3.90 (1H, m), 6.88 (1H, d, J=7Hz), 7.02 (1H, d, J=7Hz), 7.08-7.36 (5H, m), 9.87 (2H, br. s).

### Method B

To a solution of (±)*trans*-1-(3,4-dichlorobenzyl)-2-(N-methyl-N-*tert*-butoxycarbonylamino)indane (550 mg, 1.4 mmol) in dichloromethane (10ml) at 0°C was added trifluoroacetic acid (2ml) dropwise. The solution was then stirred at room temperature for 3 hours, poured into saturated aqueous NaHCO₃ (150ml) and extracted with dichloromethane (3x30ml). The combined organic phases were dried over Na₂SO₄, and concentrated *in vacuo.* The residue was subjected to column chromatography on silica eluting with 5% ethanol in chloroform to afford the pure title compound as a colourless oil (356mg).

### Example 6

### (±)trans-1-(3,4-Dichlorobenzyl)-2-dimethylaminoindane Hydrochloride (E6)

To a solution of (±)*trans*-1-(3,4-dichlorobenzyl)-2-methylaminoindane (340mg, 1.1 mmol) in acetonitrile (5ml) was added a 37% aqueous solution of formaldehyde (500µl) followed by sodium cyanoborohydride (125mg, 2mmol). The reaction was then stirred at room temperature for 1.5 h. The pH of the reaction was maintained close to neutral during this time by occasional addition of glacial acetic acid. The reaction was diluted with diethyl ether and washed with 5% aqueous sodium hydroxide. After drying over sodium sulfate, solvents were removed *in vacuo* and the residue subjected to column chromatography on silica gel eluting with 5% ethanol in chloroform to afford a colourless oil (291mg) which was converted to the HCl salt and crystallised to afford the **title compound** as a white solid. m.p. 187-189°C (from acetone/diethyl ether).
¹H Nmr (DMSO-d₆) 2.41 + 2.44 (6H, br. s + br. s, protomers), 2.82 (1H, dd, J=6, 13Hz), 3.02 (1H, dd, J=6, 13Hz), 3.20 (2H, d, J=6Hz), 3.80 (1H, m), 3.91 (1H, t, J=7Hz), 6.89 (1H, d, J=7Hz), 7.14 (4H, m), 7.49 (2H, d, J=7Hz), 11.30 (1H, br. s).

### Example 7

### (±)trans-1-(4-Benzylbenzyl)-2-methylaminoindane Hydrochloride (E7)

The title compound was prepared in a similar manner to Example 1 from LiAlH₄ (551mg, 14.5mmol), (±)*trans*-1-(4-benzylbenzyl)-2-*tert*-butoxycarbonylaminoindane (600mg, 1.45mmol) and tetrahydrofuran (40ml). After a reaction time of 2.5 h the reaction was worked up as previously described and subjected to column chromatography on silica gel eluting with 5% ethanol in chloroform to afford a colourless oil (435mg) which was converted to the HCl salt and crystallised to afford the **title compound** as a white solid. m.p. 153.5-155°C (from methanol-diethyl ether).
¹H Nmr (CDCl₃) 2.31 (3H, br. s), 2.94 (1H, dd, J=7, 14Hz), 3.07 (1H, dd, J=7, 14Hz), 3.18 (2H, m), 3.57 (1H, m), 3.83 (1H, m), 3.90 (2H, s), 6.94-7.31 (13H, m), 9.59 (1H, br. s), 9.71 (lH, br. s).

### Example 8

### (±)trans-1-(4-Benzylbenzyl)-2-dimethylaminoindane Hydrochloride (E8)

The title compound was prepared in a similar manner to Example 1 from LiAlH₄ (403mg, 10.06mmol), (±)*trans*-1-(4-benzylbenzyl)-2-(N-methyl-N-*tert*-butoxycarbonylamino)indane (454mg, 1.06mmol) and tetrahydrofuran (40ml). After a reaction time of 4 h the reaction was worked up as previously described and subjected to column chromatography on silica gel eluting with 5% ethanol in chloroform to afford a colourless oil (330mg) which was converted to the HCl salt and crystallised to afford the **title compound** as a white solid. m.p. 141-142.5°C (from methanol-diethyl ether).
¹H Nmr (DMSO-d₆) 2.45 (6H, br. s), 2.90 (1H, dd, J=7, 15Hz), 3.06 (1H, dd, J=7, 15Hz), 3.32 (2H, d J=7Hz), 3.94 (2H, m), 4.00 (2H, s), 7.03 (1H, d, J=7Hz) 7.12-7.43 (12H, m), 11.27 (1H, br. s)

### Example 9

### (±)trans-1-(2-Benzylbenzyl)-2-(N-methyl-N-pentylamino)indane Hydrochloride (E9)

The title compound was prepared in a similar manner to Example 1 from LiAlH₄ (456mg, 12mmol), (±)*trans*-1-(2-benzylbenzyl)-2-(N-pentyl-N-*tert*-butoxycarbonylamino)indane (600mg, 1.2 mmol) and tetrahydrofuran (50ml). After a reaction time of 8 h the reaction was worked up as previously described and subjected to column chromatography on silica gel eluting with 5% ethanol in chloroform to afford a colourless oil (458mg) which was converted to the HCI salt and crystallised to afford the **title compound** as a white solid. m.p. 55-66°C (from acetone-diethyl ether).
¹H Nmr of free base (CDCl₃) δ : 0.84 (3H, t, J=7Hz), 1.08-1.41 (6H, m), 1.97 (3H, s), 2.17 (2H, m), 2.88 (3H, m), 3.09 (1H, dd, J=7, 16Hz), 3.42 (2H, m), 3.93 (2H, s), 6.79 (1H, d, J=7Hz), 7.01-7.29 (12H, m).

### Example 10

### (±)trans-1-(4-Benzyloxybenzyl)-2-methylaminoindane Hydrochloride (E10)

The title compound was prepared in a similar manner to Example 1 from LiAlH₄ (722mg, 19mmol), (±)*trans*-1-(4-benzyloxybenzyl)-2-*tert*-butoxycarbonylaminoindane (600mg, 1.2 mmol) and tetrahydrofuran (40ml). After a reaction time of 2 h the reaction was worked up as previously described and subjected to column chromatography on silica gel eluting with 2% ethanol in chloroform to afford a colourless oil (581mg) which was converted to the HCI salt and crystallised to afford the **title compound** as a white solid. m.p. 172.5-173.5°C (from acetone-diethyl ether).
¹H Nmr (DMSO-d₆) δ: 2.49 (3H, s), 2.90 (1H, dd, J=7, 15Hz), 3.13 (2H, m), 3.32 (1H, dd, J=7, 15Hz), 3.67 (1H, m), 3.78 (1H, m), 5.16 (2H, s), 6.90-7.60 (13H, m), 9.40 (2H, br. s).

### Example 11

### (±)trans-1-(4-Benzyloxybenzyl)-2-dimethylaminoindane Hydrochloride (E11)

The title compound was prepared in a similar manner to Example 1 from LiAlH₄ (380mg, 10mmol), (±)*trans*-1-(4-benzyloxybenzyl)-2-(N-methyl-N-*tert*-butoxycarbonylamino) indane (470mg, 1.06mmol) and tetrahydrofuran (40ml). After a reaction time of 2 h the reaction was worked up as previously described and subjected to column chromatography on silica gel eluting with 2% ethanol in chloroform to afford a colourless oil (307mg) which was converted to the HCI salt and crystallised to afford the title compound as a white solid. m.p. 179-181°C (from methanol-diethyl ether).
¹H Nmr (DMSO-d₆) δ : 2.50+2.60 (6H, br. s+br. s, protomers), 2.91 (1H, dd, J=7, 15Hz), 3.05 (1H, dd, J=7, 15Hz), 3.32 (2H, d, J=6Hz), 3.92 (2H, m), 5.19 (2H, s), 6.92-7.62 (13H, m), 11.30 (1H, br. s)

### Example 12

### (±)trans-1-(4-Benzyloxybenzyl)-2-ethylaminoindane Hydrochloride (E12)

To a solution of (±)*trans*-1-(4-benzyloxybenzyl)-2-(N-ethyl-N-*tert*-butoxycarbonylamino) indane (300 mg) in dichloromethane (10 ml) was added trifluoroacetic acid (2 ml) dropwise. After stirring at room temperature for 2 h the reaction mixture was poured into saturated aqueous sodium bicarbonate and extracted with dichloromethane. After drying over sodium sulfate, solvents were removed *in vacuo* to afford a colourless oil which was converted to the HCl salt and crystallised to afford the **title compound** as a white solid. m.p. 188-191°C (from methanol-diethyl ether).
¹H Nmr (DMSO-d₆) δ : 1.13 (3H, t, J=7 Hz), 2.86 (3H, m), 3.08 (2H, m), 3.26 (1H, dd, J=7, 16 Hz, 3.67 (2H, m), 5.09 (2H, s), 6.85-7.60 (13H, m), 9.20 (2H, m).

### Example 13

### (±)cis-1-(4-Benzyloxybenzyl)-2-aminoindane (E13)

To a solution of lithium aluminium hydride (106mg, 2.8 mmol) in dry diethyl ether (10ml) was added dropwise a solution of (±) E, Z 1-(4-benzyloxybenzyl)-2-indanone oxime-O-methyl ether (500mg, 1.4mmol) in diethyl ether (10ml). The mixture was allowed to stir a room temperature for 18 h before careful addition of a minimum amount of water to quench the reaction. The precipitated aluminum salts were filtered off and solvents removed *in vacuo.* The residue was subjected to column chromatography on silica gel eluting with 5% ethanol in choroform. Pooling of pure fractions containing the major faster running component afforded the **title compound** as an oil (258mg).
¹H Nmr (CDCl₃) δ: 1.87 (2H, br. s), 2.75 (1H, dd, J=5, 16 Hz), 2.96 (2H, m), 3.12 (1H, dd, J=6, 15 Hz), 3.61 (1H, q, J=7Hz), 3.78 (1H, m), 5.07 (2H, s), 6.81-7.50 (13H, m).

### Example 14

### (±)cis-1-(4-Benzyloxybenzyl)-2-methylaminoindane Hydrochloride (E14)

The title compound was prepared in a similar manner to Example 1 from LiAlH₄ (292mg, 7.7mmol), (±)*cis*-1-(4-benzyloxybenzyl)-2-*tert*-butoxycarbonylaminoindane (330mg, 0.77 mmol) and tetrahydrofuran (40ml). After a reaction time of 2 h the reaction was worked up as previously described and subjected to column chromatography on silica gel eluting with 2% ethanol in chloroform to afford a colourless oil (581mg) which was converted to the HCl salt and crystallised to afford the **title compound** as a white solid. m.p. >220°C dec. (from methanol-diethyl ether).
¹H Nmr (DMSO-d₆) δ : 2.36 (1H, t, J=13Hz), 2.70 (3H, s), 3.20 (3H, m), 3.56 (1H, m), 3.93 (1H, m), 5.10 (2H, s), 6.11 (1H, d, J=7Hz), 6.87-7.51 (12H, m), 9.41 (1H, br. s), 9.63 (1H, br. s)

### Example 15

### (±)cis-1-[3-(4-Benzyloxyphenyl)propyl]-2-aminoindane (E15)

The title compound was prepared in a similar manner to Example 13 from (±) 1-[3-(4-benzyloxyphenyl)propyl]-2-indanone oxime-O-methyl ether (700 mg, 1.8mmol), lithium aluminium hydride (273mg, 7.2mmol) and diethyl ether (40ml). Column chromatography on silica gel eluting with 5% diethyl ether in hexanes afforded the **title compound** as a pale yellow oil (341mg).
¹H Nmr (CDCl₃) δ : 1.41 (2H, br. s), 1.74 94H, m), 2.71 (3H, m), 3.07 (2H, m), 3.76 (1H, q, J=5 Hz), 5.05 (2H, m), 6.90 (2H, d, J=9 Hz), 7.07-7.49 (11H, m).

### Example 16

### (±)cis-1-[3-(4-Benzyloxyphenyl)propyl]-2-methylaminoindane Hydrochloride (E16)

The title compound was prepared in a similar manner to Example 1 from LiAlH₄ (190mg, 5 mmol), (±)*cis*-1-[3-(4-benzyloxyphenyl)propyl]-2-ethoxycarbonylaminoindane (440mg, 1.0 mmol) and tetrahydrofuran (35ml). After a reaction time of 1.5 h the reaction was worked up as previously described and subjected to column chromatography on silica gel eluting with 2% ethanol in chloroform to afford a colourless oil (304mg) which was converted to the HCl salt and crystallised to afford the **title compound** as a white solid. m.p. 193-194 °C (from methanol-diethyl ether).
¹H Nmr (DMSO-d₆) δ : 1.46 (1H, m), 1.67 (3H, m), 2.55 (3H, m), 2.62 (3H, s), 3.12 (2H, m), 3.85 (1H, q, J=7 Hz), 5.08 (2H, s), 6.90 (2H, d, J=8 Hz), 7.06 (2H, d, J=8 Hz), 7.08-7.55 (9H, m), 9.24 (2H, br. s).

### Example 17

### (±) trans 1-[(4-Benzyl)benzyl]-2-aminoindane Hydrochloride (E17)

To a solution of (±) trans 1-[(4-benzyl)benzyl]-2-*tert*-butoxycarbonylaminoindane (1.10g, 2.67mmol) in dichloromethane (20ml) at 0°C was added trifluoroacetic acid (4ml) dropwise. The solution was then stirred at room temperature for 2 h, poured into saturated aqueous NaHCO₃ (150ml) and extracted with dichloromethane (3x30ml). The combined organic phases were dried over Na₂SO₄, and concentrated *in vacuo.* The residue was subjected to column chromatography on silica eluting with 5% ethanol in chloroform to afford a colourless oil (794mg) which was converted to the HCl salt and crystallised to afford the **title compound** as a white solid. m.p. 202-204°C. (from methanol-diethyl ether).
¹H Nmr (DMSO-d₆) δ : 2.88 (1H, dd, J=7,15Hz), 2.99 (1H, dd, J=3,16Hz), 3.14 (1H, dd, J=5,15Hz), 3.33 (1H, dd, J=7,16Hz), 3.69 (2H, m), 4.01 (2H, s), 7.09 (1H, d, J=8Hz), 7.32 (12H, m), 8.48 (3H, br. s).

### Example 18

### (±) cis-1-(4-Methoxybenzyl)-2-methylaminoindane Hydrochloride (E18)

The title compound was prepared in a similar manner to Example 1 from LiAlH₄ (277mg, 7.30mmol), (±) *cis*-1-(4-methoxybenzyl)-2-ethoxycarbonylaminoindane (430mg, 1.32 mmol) and tetrahydrofuran (50ml). After a reaction time of 1.5h the reaction was worked up as previously described and subjected to column chromatography on silica gel eluting with 5% ethanol in chloroform to afford a colourless oil (307mg) which was converted to the HCl salt and crystallised to afford the **title compound** as a white solid. m.p. >252°C dec. (from methanol-diethyl ether).
¹H Nmr (DMSO-d₆) δ :2.37 (1H, t, J=14Hz), 2.70 (3H, s), 3.10-3.64 (4H, m), 3.75 (3H, s), 3.95 (1H, m), 6.14 (1H, d, J=8Hz), 6.94 (5H, m), 7.16 (1H, t, J=7Hz), 7.29 (1H, d, J=7Hz), 9.50 (2H, br.s)

### Example 19

### (±) cis-1-[(4-Benzyl)phenoxymethyl]-2-aminoindane Hydrochloride (E19)

To a solution of (±) cis-1-[(4-benzoyl)phenoxymethyl]-2-aminoindane (2.83g, 8.25mmol) in dichloromethane (20ml) and trifluoroacetic acid (75ml) at 0°C under argon was added sodium borohydride pellets (1.89g, 50mmol) over 1 hour. The cooling bath was then removed and stirring continued at room temperature for 16 h. The reaction mixture was then poured into water (1000ml), basified with solid potassium carbonate and then extracted with diethyl ether (3 x 200ml). The combined organic extracts were dried over sodium sulfate and concentrated *in vacuo.* The residue was subjected to column chromatography on silica gel eluting with 7% ethanol in chloroform to afford a colourless oil (2.1g) which was converted to the HCl salt and crystallised to afford the **title compound** as a white solid. m.p. 143-145 °C (from methanol-diethyl ether).
¹H Nmr (DMSO-d₆) δ : 3.12 (1H, dd, J=5, 15Hz), 3.43 (1H, m), 3.79 (1H, m), 3.98 (3H, m), 4.37 (2H, m), 7.00 (2H, d, J=8Hz), 7.13-7.58 (11H, m), 8.59 (3H, br. s).

### Example 20 (±)cis-1-[(4-Benzyl)phenoxymethyl]-2-methylaminoindane Hydrochloride (E20)

The title compound was prepared in a similar manner to Example 1 from LiAlH₄ (490mg, 13 mmol), (±)cis-1-[(4-benzyl)phenoxymethyl]-2-*tert*-butoxycarbonylaminoindane (1.11g, 2.6 mmol) and tetrahydrofuran (50ml). After a reaction time of 3 h the reaction was worked up as previously described and subjected to column chromatography on silica gel eluting with 5% ethanol in chloroform to afford a colourless oil (877mg) which was converted to the HCI salt and crystallised to afford the **title compound** as a white solid. m.p. 155-156 °C (from methanol-diethyl ether).
¹H Nmr (DMSO-d₆) δ : 2.63 (3H, s), 3.17 (1H, dd, J=6, 18Hz), 3.36 (1H, m), 3.86 (4H, m), 4.30 (2H, m), 6.90 (2H, d, J=7Hz), 7.22 (10H, m),7.39 (1H, m), 9.45 (2H, br. s).

### Example 21

### (±)cis-1-(4-Phenoxybenzyl)-2-methylaminoindane Hydrochloride (E21)

The title compound was prepared in a similar manner to Example 1 from LiAlH₄ (257mg, 6.77mmol), (±)*cis*-1-(4-phenoxybenzyl)-2-*tert*-butoxycarbonylaminoindane (483mg, 1.16. mmol) and tetrahydrofuran (50ml). After a reaction time of 2h the reaction was worked up as previously described and subjected to column chromatography on silica gel eluting with 5% ethanol in chloroform to afford a colourless oil which was converted to the HCl salt and crystallised to afford the **title compound** as a white solid. m.p. 167-169°C dec. (from ethanol-diethyl ether).
¹H Nmr (DMSO-d₆) δ :2.45 (1H, t, J=12Hz), 2.72 (3H, s), 3.19-3.40 (3H, m), 3.60 (1H, m), 3.97 (1H, q, J=7Hz), 6.19(1H, d, J=7Hz), 6.95-7.20 (9H, m), 7.29 (1H, d, J=7Hz), 7.40 (2H, t, J=7Hz), 9.58 (2H, br.s)

### Example 22

### (±)cis-1-(3,4-Dichlorobenzyl)-2-aminoindane Hydrochloride (E22)

To a solution of lithium aluminium hydride (2.19g, 58 mmol) in dry diethyl ether (100ml) was added dropwise a solution of (±) E,Z-1-(3,4-dichlorobenzyl)-2-indanone oxime-O-methyl ether (8.04g, 25mmol) in diethyl ether (400ml). The mixture was allowed to stir at room temperature for 18 h before careful addition of a minimum amount of water to quench the reaction. The precipitated aluminum salts were filtered off and solvents removed *in vacuo.* The residue was subjected to column chromatography on silica gel eluting with 5% ethanol in choroform. Pooling of pure fractions containing the major faster running component afforded an oil (2.11g) which was converted to the HCl salt and crystallised to give the **title compound** as a white solid. m.p. >198°C dec. (from ethanol-diethyl ether).
¹H Nmr (CDCl₃) δ: 2.61 (1H, t, J=14Hz), 3.13-3.25 (3H, m), 3.63 (1H, m), 4.02 (1H, m), 6.42 (1H, d, J=7Hz), 7.03 (1H, t, J=7Hz), 7.16 (2H, m), 7.30 (1H, d, J=7Hz), 7.45 (1H, s), 7.60 (1H, d, J=7Hz), 8.57 (3H, br.s)

### Example 23

### (±)cis-1-(3,4-Dichlorobenzyl)-2-methylaminoindane Hydrochloride (E23)

To a solution of (±) *cis*-1-(3,4-dichlorobenzyl)-2-(N-methyl-N-*tert*-butoxycarbonylamino)indane (1.016g, 2.5 mmol) in dichloromethane (20ml) at 0°C was added trifluoroacetic acid (4ml) dropwise. The solution was then stirred at room temperature for 2 h, poured into saturated aqueous NaHCO₃ (150ml) and extracted with dichloromethane (3x30ml). The combined organic phases were dried over Na₂SO₄, and concentrated *in vacuo.* The residue was subjected to column chromatography on silica eluting with 5% ethanol in chloroform to afford a colourless oil (585mg) which was converted to the HCl salt and crystallised to afford the **title compound** as a white solid. m.p. >245°C dec. (from ethanol-diethyl ether).
¹H Nmr (CDCl₃) δ : 2.48 (1H, t, J=14Hz), 2.72 (3H, s), 3.23 (3H, m), 3.61 (1H, m), 3.94 (1H, q, J=7Hz), 6.21 (1H, d, J=7Hz), 7.00 (1H, t, J=7Hz), 7.09 (1H, d, J=7Hz), 7.19 (1H, t, J=7Hz), 7.30 (1H, d, J=7Hz), 7.38 (1H, s), 7.58 (1H, d, J=7Hz), 9.66 (2H, br.s)

### Examples 24 and 25

### (±)cis-1-(4-Benzyloxybenzyl)-2-methylaminoindane Hydrochloride (E24)

### (-)cis-1-(4-Benzyloxybenzyl)-2-methylaminoindane Hydrochloride (E25)

To a solution of S-(+)-α-methoxyphenylacetic acid (2.03g, 12.2mmol) in dichloromethane (100ml) was added thionyl chloride (15ml) and the mixture heated at reflux for 90 minutes. On cooling, the mixture was concentrated *in vacuo.* The residue was then dissolved in dichloromethane (50ml) and added to a mixture of (±)*cis*-1-(4-benzyloxybenzyl)-2-methylaminoindane (4.2g, 12.2mmol), dichloromethane (50ml) and 1M aqueous sodium hydroxide (100ml). The two phase mixture was then stirred vigorously for lh. The layers were then separated and the aqueous phase extracted with dichloromethane. The combined organic phases were washed with brine, dried over sodium sulfate and concentrated in *vacuo* to afford a 50:50 mixture of two diastereomers which were readily separated by column chromatography on silica gel, eluting with 50% ether in 60/80 petrol.
The less polar diastereomer (2.7g) was then dissolved in dry tetrahydrofuran (150ml) and treated with potassium tert-butoxide (27g), and water (450µl). The mixture was then stirred vigorously for 90 minutes. After filtering off the solid material, the mixture was poured into water (400ml) and extracted with ether. The combined organic extracts were dried over sodium sulfate and concentrated *in vacuo* to afford a yellow oil which was converted to the HCI salt and crystallised to afford **(+) *cis*-1-(4-benzyloxybenzyl)-2-methylaminoindane hydrochloride (E24)** (1.58g) as a white solid. m.p. >225 °C dec. (from methanol-diethyl ether).
Specific optical rotation = +110° (chloroform, c=1, 20°C)

In a similar manner, the more polar diastereomer (2.6g) afforded **(-)*cis*-1-(4-benzyloxybenzyl)-2-methylaminoindane hydrochloride (E25)** (1.30g) as a white solid. m.p. >225°C dec.
Specific optical rotation = -117° (chloroform, c=1, 20°C)

### Example 26

### (±)cis-1-(4-Trifluoromethylbenzyl)-2-aminoindane Hydrochloride (E26)

To a solution of lithium aluminium hydride (1.81g, 48 mmol) in dry diethyl ether (100ml) was added dropwise a solution of (±) E,Z 1-(4-trifluoromethylbenzyl)-2-indanone oxime-O-methyl ether (6.24g, 20mmol) in diethyl ether (400ml). The mixture was allowed to stir at room temperature for 18 h before careful addition of a minimum amount of water to quench the reaction. The precipitated aluminum salts were filtered off and solvents removed *in vacuo.* The residue was subjected to column chromatography on silica gel eluting with 5% ethanol in choroform. Pooling of pure fractions containing the major faster running component afforded an oil (1.74g) which was converted to the HCI salt and crystallised to give the **title compound** as a white solid. m.p. >210°C dec. (from ethyl acetate-diethyl ether-60/80 petrol).
¹H Nmr (CDCl₃) δ : 2.70 (1H, t, J=14Hz), 3.17 (3H, m), 3.68 (1H, m), 4.06 (1H, q, J=7Hz), 6.32 (1H, d, J=7Hz), 7.00 (1H, t, J=7Hz), 7.18 (1H, t, J=7Hz), 7.31 (1H, d, J=7Hz), 7.40 (2H, d, J=7Hz), 7.70 (2H, d, J=7Hz), 8.56 (3H, br.s)

### Example 27

### (±)cis-1-(4-Methoxybenzyl)-2-aminoindane (E27)

The title compound was prepared in a similar manner to Example 13 from (±) E,Z-1-(4-methoxybenzyl)-2-indanone oxime-O-methyl ether (2.48g, 8.4mmol), lithium aluminium hydride (0.66g, 17mmol) and diethyl ether (150ml). Column chromatography on silica gel eluting with 5% ethanol in chloroform afforded the **title compound** as a pale yellow oil (0.92g).
¹H Nmr (CDCl₃) δ : 1.50 (2H, br.s), 2.70 (1H, dd, J=5,15Hz), 2.96 (2H, d, J=7Hz), 3.10 (1H, dd, J=7,15Hz), 3.35-3.44 (1H, m), 3.71-3.78 (1H, m), 3.82 (3H, s), 6.88 (3H, t, J=9Hz), 7.07-7.23 (5H, m).

### Example 28

### (±)cis-1-(4-Phenoxybenzyl)-2-aminoindane (E28)

The title compound was prepared in a similar manner to Example 13 from (±) E,Z-1-(4-phenoxybenzyl)-2-indanone oxime-O-methyl ether (6.30g, 18mmol), lithium aluminium hydride (1.48g, 40mmol) and diethyl ether (350ml). Column chromatography on silica gel eluting with 5% ethanol in chloroform afforded the **title compound** as a pale yellow oil (1.01g).
¹H Nmr (CDCl₃) δ : 1.46 (2H, br.s), 2.71 (1H, dd, J=5,15Hz), 2.95-3.15 (3H, m), 3.36-3.45 (1H, m), 3.74-3.80 (1H, m), 6.89-7.38 (13H, m).

### Example 29

### (±)cis-1-[(4-Benzoyl)phenoxymethyl]-2-aminoindane (E29)

A solution of (±) cis-1-hydroxymethyl-2-aminoindane (1.5g, 9.2mmol) in dry dimethyl sulfoxide (30ml) was treated with sodium hydride (330mg of an 80% dispersion in oil, 11mmol). After stirring for lh at room temperature under argon, 4-fluorobenzophenone (2.21g, 11mmol) was added in one portion and stirring continued overnight at room temperature. The mixture was then poured into water (200ml) and extracted three times with ether. After drying over sodium sulfate, volatiles were removed *in vacuo* and the residue subjected to column chromatography on silica gel eluting with 5% ethanol in chloroform to afford the **title compound** as a brown oil (2.45g).
¹H Nmr (CDCl₃) δ : 1.68 (2H, br. s), 2.76 (1H, dd, J=6,16Hz),3.33 (2H, m), 3.74 (1H,q, J=6Hz), 4.20 (1H, t, J=7Hz), 4.39 (1H, dd, J=5,7Hz), 6.90 (2H, d, J=8Hz), 7.25 (4H, m), 7.52 (3H, m), 7.75 (2H, d, J=7Hz), 7.82 (2H, d, J=7Hz).

### Example 30

### (±) cis-1-(4-Benzyloxybenzyl)-2-dimethylaminoindane Hydrochloride (E311)

The title compound was prepared in a similar manner to Example 1 from LiAlH₄ (159mg, 4mmol), (±) *cis*-1-(4-benzyloxybenzyl)-2-(N-methyl-N-*tert*-butoxycarbonylaminoindane (370mg, 0.8 mmol) and tetrahydrofuran (40ml). After a reaction time of 2.5h the reaction was worked up as previously described and subjected to column chromatography on silica gel eluting with 5% ethanol in chloroform to afford a colourless oil (270mg) which was converted to the HCI salt and crystallised to afford the **title compound** as a white solid. m.p. >210°C dec. (from methanol-diethyl ether).
¹H Nmr (DMSO-d₆) δ : 2.37 (1H, t, J=8Hz), 2.88 (3H, br. s), 3.01 (3H, br. s), 3.05 (1H, m), 3.27 (2H, m), 3.56 (1H, m), 4.00 (1H, m), 5.06 (2H, s), 6.10 (1H, d, J=8Hz), 6.92(4H, m), 7.16 (1H, t, J=7Hz), 7.28 (1H, d, J=7Hz), 7.45 (6H, m), 10.70 (1H, br. s).

### Example 31

### (±)-1-[(4-Cyclopropylmethoxy)benzyl)]-2-aminoindane Hydrochloride (E31)

The title compound was prepared in a similar manner to Example 13 from (±) E,Z-1-[(4-cyclopropylmethoxy)benzyl)]-2-indanone oxime-O-methyl ether (1.2g, 3.7mmol), lithium aluminium hydride (426mg, 11mmol) and diethyl ether (70ml). Column chromatography on silica gel eluting with 5% ethanol in chloroform afforded a colourless oil (594mg) which was converted to the HCl salt and crystallised to afford the **title compound** as a white solid. m.p. >240°C dec. (from methanol-diethyl ether).
¹H Nmr (CDCl₃) δ : 0.34 (2H, m), 0.57 (2H, m), 1.22 (1H, m), 2.50 (1H, m), 3.12 (3H, m), 3.58 (1H, m), 3.80 (2H, m), 4.03 (1H, q, J=7Hz), 6.36 (1H, d, J=7Hz), 6.87 (2H, d, J=8Hz), 6.97 (1H, t, J=7Hz), 7.05 (2H, d, J=8Hz), 7.16 (1H, t, J=7Hz), 7.28 (1H, d, J=7Hz), 8.49 (3H, br. s).

### Example 32

### (±) cis-1-(3,4-Dichlorophenoxymethyl)-2-aminoindane Hydrochloride (E32)

A solution of (±) cis-1-hydroxymethyl-2-aminoindane (1.0g, 6.1mmol) in dry dimethyl sulfoxide (25ml) was treated with sodium hydride (221mg of an 80% dispersion in oil, 7.3mmol). After stirring for lh at room temperature under argon, 1,2-dichloro-4-fluorobenzene (0.86ml, 7.3mmol) was added in one portion and stirring continued overnight at room temperature. The mixture was then poured into water (200ml) and extracted three times with diethyl ether. After drying over sodium sulfate, volatiles were removed *in vacuo* and the residue subjected to column chromatography on silica gel eluting with 5% ethanol in chloroform to afford a brown oil (501mg) which was converted to the HCl salt and crystallised to afford the **title compound** as a white solid. m.p. 235-238°C dec. (from methanol-diethyl ether).
¹H Nmr (CDCl₃) δ : 3.05 (1H, dd, J=6,15Hz), 3.40 (1H, dd, J=8,15Hz), 3.73 (1H, m), 3.92 (1H, m), 4.40 (2H, d, J=6Hz), 7.05 (1H, dd, J=3,9Hz), 7.34 (5H, m), 7.54 (1H, d, J=9Hz), 8.62 (3H, br. s).

### Example 33

### (±)cis-1-(4-n-Butoxybenzyl)-2-aminoindane Hydrochloride (E33)

The title compound was prepared in a similar manner to Example 13 from (±) E,Z-1-(4-n-butoxybenzyl)-2-indanone oxime-O-methyl ether (1.48g, 4.6mmol), lithium aluminium hydride (522mg, 13.7mmol) and diethyl ether (60ml). Column chromatography on silica gel eluting with 5% ethanol in chloroform afforded a colourless oil (609mg) which was converted to the HCl salt and crystallised to afford the **title compound** as a white solid. m.p. >205°C dec. (from methanol-diethyl ether).
¹H Nmr (CDCl₃) δ: 0.92 (3H, t, J=7Hz), 1.45 (2H, m), 1.70 (2H, m), 2.51 (1H, m), 3.11 (3H, m), 3.59 (1H, m), 3.97 (3H, m), 6.37 (lH, d, J=7Hz), 6.88 (2H, d, J=8Hz), 6.98 (1H, t, J=7Hz), 7.07 (2H,d, J=8Hz), 7.17 (1H, t, J=7Hz), 7.29 (1H, d, J=7Hz), 8.45 (3H, br. s).

### Example 34

### (±)cis-1-(3,4-Dimethoxybenzyl)-2-aminoindane Hydrochloride (E34)

The title compound was prepared in a similar manner to Example 13 from (±) E,Z-1-(3,4-dimethoxybenzyl)-2-indanone oxime-O-methyl ether (2.5g, 8mmol), lithium aluminium hydride (672mg, 24mmol) and diethyl ether (85ml). Column chromatography on silica gel eluting with 5% ethanol in chloroform afforded a colourless oil (742mg) which was converted to the HCl salt and crystallised to afford the **title compound** as a white solid. m.p. >205°C dec. (from methanol-diethyl ether).
¹H Nmr (CDCl₃) δ : 2.50 (1H, m), 3.13 (3H, m), 3.62 (1H, m), 3.72 (3H, s), 3.77 (3H, s), 4.03 (1H, q, J=7Hz), 6.44 (1H, d, J=7Hz), 6.69 (1H, dd, J=1,7Hz), 6.75 (1H, d, J=1Hz), 6.90 (1H, d, J=7Hz), 7.00 (1H, t, J=7Hz), 7.18 (1H, t, J=7Hz), 7.30 (1H, d, J=7Hz), 8.54 (3H, br. s)

### Example 35

### (±)cis-2-Amino-1-[(5-chloro-2-thienyl)methyl]indane Maleate (E35a) and

### (±)trans-2-Amino-1-[(5-chloro-2-thienyl)methyl]indane Hydrochloride (E35b)

To a suspension of lithium aluminium hydride (1.40g, 37 mmol) in dry diethyl ether (50ml) was added dropwise a solution of (±) E,Z 1-[(5-chloro-2-thienyl)methyl]-2-indanone oxime-O-methyl ether (3.60g, 12mmol) in diethyl ether (75ml). The mixture was allowed to stir at room temperature for 6 h and then worked up as described in Example 13 to give a dark oil (3.2g) which was subjected to column chromatography on silica gel eluting with 0-4% ethanol in choroform. Pooling of pure fractions containing the major faster running component afforded an oil (1.0g) which was converted into the maleate salt and crystallised to give the **title compound** (**E35a**) as a white solid. m.p. 143-145 °C (from methanol-diethyl ether).
¹H Nmr (DMSO-d₆) δ : 2.85-3.30 (4H, m), 3.60 (1H, m), 4.10 (1H, q, J=6Hz), 6.02 (2H, s, maleate signal) 6.73 (2H, m), 7.00 (1H, d, J=4Hz), 7.10 (1H, t, J=7Hz), 7.21 (1H, t, J=7Hz), 7.32 (1H, d, J=7Hz), 8.10 (3H, br. s)

The minor slower running component was isolated as an oil (0.14g) which was converted into the hydrochloride salt and crystallised to give the **title compound** (**E35b**) as a white solid m.p. 200-201 °C (dec) (from methanol-diethyl ether).
¹H Nmr (DMSO-d₆) δ : 2.92 (1H, dd, J=4, 15Hz), 3.10-3.45 (3H, m), 3.60 (2H, m), 6.80 (1H, d, J=3Hz), 6.90 (1H, d, J=3Hz), 7.20 (4H, m), 8.55 (3H, br. s)

### Example 36

### (±)cis-1-[(5-Chloro-2-thienyl)methyl]-2-methylaminoindane Hydrochloride (E36)

A solution of (±) cis-l-[(5-chloro-2-thienyl)methyl]-2-(N-methyl-N-*tert*-butoxycarbonylamino)indane (0.70g, 1.85mmol) in dry dichloromethane (30ml) was cooled in ice and treated with trifluoroacetic acid (3ml). The mixture was stirred at room temperature for 2h and then poured into saturated aqueous sodium hydrogen carbonate (150ml). The aqueous layer was extracted with dichloromethane (3x75ml) and the combined organic phases were dried over sodium sulfate and concentrated *in vacuo.* Purification on silica gel eluting with 0-5% ethanol in chloroform afforded a yellow oil (0.5g) which was extracted into hexane. Insoluble impurities were removed by filtration. The filtrate was treated with ethereal HCl to give the hydrochloride salt. Crystallisation afforded the **title compound** as a white solid m.p. 224-226 °C (dec) (from methanol-diethyl ether).
¹H Nmr (DMSO-d₆) δ : 2.58-2.82 (4H, m), 3.15-3.40 (3H, m), 3.56 (1H, m), 3.94 (1H, q, J=7Hz), 6.50 (1H, d, J=7Hz), 6.58 (1H, d, J=3Hz), 6.95 (1H, d, J=3Hz), 7.03 (1H, t, J=7Hz), 7.20 (1H, t, J=7Hz), 7.28 (1H, d, J=7Hz)

### Example 37

### (±)cis-2-Cyclopropylmethylamino-1-(3,4-dichlorobenzyl)indane Hydrochloride (E37)

A solution of (±) *cis*-1-(3,4-dichlorobenzyl)-2-(N-cyclopropylmethyl-N-*tert*-butoxycarbonylamino)indane (0.7g, 2mmol) in dry dichloromethane (15ml) was cooled in ice and treated with trifluoroacetic acid (3ml). The mixture was stirred at room temperature for 3h and then poured into saturated aqueous sodium hydrogen carbonate (150ml). The aqueous layer was extracted with dichloromethane (3x75ml) and the combined organic phases were dried over sodium sulfate and concentrated *in vacuo.* Purification on silica gel eluting with 5% ethanol in chloroform afforded a yellow oil (0.5g). The oil was treated with ethereal HCl to give the hydrochloride salt Crystallisation afforded the **title compound** as a white solid m.p. > 280°C dec. (from methanol-diethyl ether).
¹H Nmr (DMSO-d₆) δ : 0.47 (2H, m), 0.63 (2H, m), 1.23 (1H, m), 2.40 (1H, m), 3.02 (2H, m), 3.19-3.26 (3H, m), 3.60 (1H, m), 4.02 (1H, m), 6.17 (1H, d, J=7Hz), 6.98 (1H, t, J=7Hz), 7.07 (1H, dd, J=1,7Hz), 7.18 (1H, t, J=7Hz), 7.30 (1H, d, J=7Hz), 7.40 (1H, d, J=1Hz), 7.56 (1H, d, J=7Hz), 9.46 (2H, br. s).

### Example 38

### (±)cis-1-(3,4-Dichlorobenzyl)-2-dimethylaminoindane Hydrochloride (E38)

A solution of (±)*cis*-1-(3,4-dichlorobenzyl)-2-aminoindane (1.09g, 3.7mmol) in acetonitrile (50ml) was treated with formaldehyde (2.5ml of a 37% solution, 33mmol) and sodium cyanoborohydride (0.47g, 7.5mmol). The pH was adjusted to 5-7 by addition of glacial acetic acid and the reaction was stirred at room temperature overnight The reaction mixture was poured into 1M aqueous NaOH (100ml) and extracted into diethyl ether (3x100ml). The combined extracts were washed with brine, dried over magnesium sulfate and concentrated *in vacuo.* The residue was subjected to column chromatography on silica gel eluting with 5% ethanol in chloroform to afford an oil (1g) which was converted to the HCl salt and crystallised to give the **title compound** as a white solid. m.p. >230°C dec. (from methanol-diethyl ether).
¹H Nmr (DMSO-d₆) : 2.91 and 3.06 (each 3H, d, J=3Hz)(protomers), 3.13-3.32 (4H, m), 3.63 (1H, m), 4.02 (1H, m), 6.13 (1H, d, J=7Hz), 6.93-7.00 (2H, m), 7.19 (1H, t, J=7Hz), 7.30 (1H, d, J=7Hz), 7.44 (1H, s), 7.52 (1H, d, J=7Hz), 10.88 (1H, br.s).

### Example 39

### (±)cis-1-(3,4-Dichlorobenzyl)-2-isopropylaminoindane Hydrochloride (E39)

A solution of (±)*cis*-1-(3,4-dichlorobenzyl)-2-aminoindane (0.49g, 1.7mmol) in acetonitrile (25ml) was treated with acetone (1.25ml 17mmol) and sodium cyanoborohydride (0.22g, 3.5mmol). The pH was maintained at 5-7 by addition of glacial acetic acid and the reaction was stirred at room temperature overnight. The mixture was poured into 1M aqueous NaOH (100ml) and extracted into diethyl ether (3x100ml). The combined extracts were washed with brine. After drying over sodium sulfate, volatiles were removed *in vacuo* and the residue subjected to column chromatography on silica gel eluting with 5% ethanol in chloroform to afford an oil (0.35g) which was converted to the HCl salt and crystallised to afford the **title compound** as a white solid. m.p. >249°C dec. (from methanol-diethyl ether).
¹H Nmr OJMSO-d₆) : 1.40 (6H, m), 2.44-2.51 (2H, m), 3.56 (2H, m), 4.11 (1H, br.s), 6.15 (1H, d, J=7Hz), 6.97 (1H, t, J=7Hz), 7.06 (1H, dd, J=1,7Hz), 7.17 (1H, t, J=7Hz), 7.29 (1H, d, J=7Hz), 7.40 (1H, s), 7.55 (1H, d, J=7Hz), 9.22 (2H, br.s), 9.67 (2H, br.s).

### Example 40

### (±) cis-1-[4-(4-fluorophenoxy)benzyl]-2-aminoindane Maleate (E40)

To a suspension of lithium aluminium hydride (0.55g, 14.5mmol) in dry diethyl ether (50ml) under argon was added dropwise a solution of (±) E,Z 1-[4-(4-fluorophenoxy)benzyl]-2-indanone oxime-O-methyl ether (1.75g, 4.85mmol) in diethyl ether (50ml). The mixture was allowed to stir at room temperature for 9h and then worked up as described in Example 13 to give an oil (1.65g) which was subjected to column chromatography on silica gel eluting with 0-4% ethanol in choroform. Pooling of pure fractions containing the major faster running component afforded an oil (0.83g) which
was converted into the maleate salt and crystallised to give the **title compound** as a white solid. m.p. 168.5-169.5°C (from methanol-diethyl ether).
¹H Nmr (DMSO-d₆) δ : 2.60 (1H, t, J=12Hz), 3.06 (2H, m), 3.20 (1H, dd, J=7, 15Hz), 3.62 (1H, m), 4.09 (1H, q, J=7Hz), 6.02 (2H, s, maleate), 6.45 (1H, d, J=7Hz), 6.92-7.32 (12H, m).

### Example 41

### (±) cis-1-[4-(4-Fluorophenoxy)benzyl]-2-methylaminoindane Hydrochloride (E41)

A solution of (±) cis-1-[4-(4-fluorophenoxy)benzyl]-2-(N-methyl-N-*tert*-butoxycarbonylamino)indane (0.16g, 0.35mmol) in dry dichloromethane (10ml) was cooled in ice and treated with trifluoroacetic acid (1ml). The mixture was stirred at room temperature for 2h and then poured into saturated aqueous sodium hydrogen carbonate (50ml). The aqueous layer was extracted with dichloromethane (3x50ml) and the combined organic phases were washed with brine, dried over sodium sulfate and concentrated *in vacuo.The* resulting pale yellow oil (0.1g) was extracted into hexane and insoluble impurities were removed by filtration. The filtrate was treated with ethereal HCI to give the hydrochloride salt. Crystallisation afforded the **title compound** as a white solid m.p. 169-171°C (from methanol-diethyl ether).
¹H Nmr (DMSO-d₆) δ: 2.42 (1H, t, J=13Hz), 2.70 (3H, s), 3.20 (3H, m), 3.58 (1H, m), 3.95 (1H, m), 6.15 (1H, d, J=7Hz), 6.90-7.32 (12H, m), 9.40 (1H, br. s), 9.70 (1H, br.s).

### Example 42

### (±) cis-1-[4-(4-Fluorophenoxy)benzyl]-2-isopropylaminoindane Hydrochloride (E42)

A solution of (±) cis-1-[4-(4-fluorophenoxy)benzyl]-2-aminoindane (0.45g, 1.35mmol) in acetonitrile (15ml) was treated with acetone (1.0ml 13.5mmol) and sodium cyanoborohydride (0.17g, 2.7mmol). The pH was adjusted to 5-7 by addition of glacial acetic acid and the reaction was stirred at room temperature overnight The reaction mixture was poured into 1M aqueous NaOH (100ml) and extracted into diethyl ether (3x100ml). The combined extracts were washed with brine, dried over sodium sulfate and concentrated *in vacuo* to give a brown foam. Column chromatography on silica gel eluting with 0-4% ethanol in chloroform afforded an oil (66mg) which was extracted into a mixture of diethyl ether and hexane, and then filtered to remove insoluble impurities. Treatment of the filtrate with ethereal HCI afforded the hydrochloride salt which was crystallised to afford the **title compound** as a white solid m.p. 236-238°C (dec.) (from methanol-diethyl ether).
¹H Nmr (DMSO-d₆) : 1.37 (6H, overlapping doublets), 2.43 (1H, t, J=13Hz), 3.20 (3H, m), 3.50 (2H, m), 4.12 (1H, m), 6.15 (1H, d, J=7Hz), 6.88-7.35 (12H, m), 9.02 (1H, br. s), 9.28 (1H, br. s).

### Example 43

### (±)cis-1-Benzyl-2-aminoindane Hydrochloride (43)

To a solution of lithium aluminium hydride (1.12g, 30mmol) in dry diethyl ether (50ml) was added dropwise a solution of (±) E,Z-1-benzyl-2-indanone oxime-o-methyl ether (3.15g, 13mmol) in diethyl ether (200ml). After stirring at room temperature for 18 h the reaction was worked up as described in Example 13. The crude product was subjected to column chromatography on silica gel eluting with 5% ethanol in choroform. Pooling of pure fractions containing the major faster running component afforded an oil (0.88g) which was converted to the HCl salt and crystallised to give the **title compound** as a white solid. m.p. >230°C (dec.) (from methanol-diethyl ether).
¹H Nmr (DMSO-d₆) δ: 2.57 (1H, d, J=12Hz), 3.15-3.22 (3H, m), 3.63 (1H, m), 4.04 (1H, m), 6.28 (1H, d, J=7Hz), 6.95 (1H, t, J=7Hz), 7.12-7.19 (3H, m), 7.21-7.37 (4H, m), 8.55 (3H, br.s).

### Example 44

### (±)cis-1-(4-Trifluoromethylbenzyl)-2-methylaminoindane Hydrochloride (E44)

To a solution of lithium aluminium hydride (524mg, 13.81mmol) in tetrahydrofuran (10ml) was added a solution of (±)*cis*-1-(4-trifluoromethylbenzyl)-2-*tert*-butoxycarbonylaminoindane (918mg, 2.35mmol) in tetrahydrofuran (50ml). The reaction mixture was heated at reflux under argon for 2 h and then worked up as described in Example 1. Purification by column chromatography on silica gel eluting with 5% ethanol in chloroform afforded an oil (0.5g).which was converted to the HCl salt and crystallised to give the **title compound** as a white solid. m.p. 229-232°C (dec.) (from methanol-diethyl ether).
¹H Nmr(DMSO-d₆) δ: 2.54 (3H, t, J=12Hz), 2.72 (3H, s), 3.27 (3H, m), 3.66 (1H, m), 3.98 (1H, q, J=7Hz), 6.09 (1H, d, J=7Hz), 6.85-7.70 (7H, m), 9.60 (3H, br.s).

## Claims

1. A compound of formula (I) : 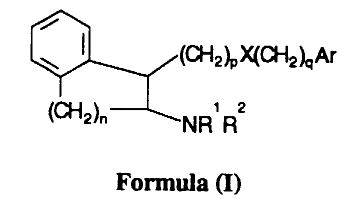 wherein
X represents 0, S, NH or a bond;
p and q independently represent 0-4 such that the sum of p+q is at least 1;
R¹ and R² each independently represent hydrogen, C₁₋₆alkyl, C₃₋₆cycloalkyl, or C₁₋₄alkyl-C₃₋₆cycloalkyl;
n is 1,2 or 3; and
Ar represents phenyl optionally substituted by 1to 3 substituents selected from :
halo, C₁₋₄alkyl, C₁₋₄alkoxy, C₁₋₂alkylenedioxy, trifluoromethyl, trifluoromethoxy, CN, NO₂, amino, mono- or di- alkylamino, optionally substituted benzoyl and Ph(CH₂)ᵣY(CH₂)ₛ- where Ph is optionally substituted phenyl, Y is oxygen or a bond and r and s each independently represent 0-4 provided that the sum of r+s is not greater than 4, or
Ar represents an optionally substituted unsaturated monocyclic heteroaryl ring system containing 5 or 6 ring members, or an optionally substituted, unsaturated or partially saturated bicyclic aryl or heteroaryl ring system containing 8-10 ring members,
or a salt thereof.

2. A compound according to claim 1 wherein Ar represents phenyl substituted by a group Ph(CH₂)ᵣY(CH₂)ₛ-.

3. A compound according to claim 2 wherein r and s independently represent zero or 1, such that the sum of r and s does not exceed 1.

4. A compound according to any of claims 1 to 3 wherein X represents a bond.

5. A compound according to any of claims 1 to 4 wherein R¹ is methyl or isopropyl.

6. A compound according to any of claims 1 to 5 wherein R² is hydrogen.

7. A compound according to claim 4 wherein the sum of p and q is from 1 to 3.

8. A compound according to any of claims 1 to 7 wherein n is 1.

9. A compound according to any of claims 1 to 8 in which the optionally substituted benzoyl group or the Ph(CH₂)ᵣY(CH₂)ₛ- group is in the 4-position.

10. A compound according to any of claims 1 to 9 wherein Ph(CH₂)ᵣY(CH₂)ₛ- is optionally substituted benzyloxy, benzyl, or phenoxy, where the optional substituents are halogen, C₁₋₄alkoxy, C₁₋₄alkyl, trifluoromethyl or trifluoromethoxy.

11. A compound according to any of claims 1 to 10 wherein the substiuents on optionally substituted benzoyl, benzyloxy, benzyl, or phenoxy is 4-fluoro, 4-chloro, 3-fluoro, 3-chloro or 3,4-dichloro.

12. A compound of claim 1 selected from :
(±)*trans*-1-(4-Benzylbenzyl)-2-methylaminoindane;
*(±)trans-*1-(4-Benzylbenzyl)-2-dimethylaminoindane;
(±)*trans*-1-(4-Benzyloxybenzyl)-2-methylaminoindane;
*(±)trans-*1-(4-Benzyloxybenzyl)-2-dimethylaminoindane;
(±)*trans*-1-(4-Benzyloxybenzyl)-2-ethylaminoindane;
(±)*cis*-1-(4-Benzyloxybenzyl)-2-aminoindane;
(±)*cis*-1-(4-Benzyloxybenzyl)-2-methylaminoindane;
(±)*cis*-1-[3-(4-Benzyloxyphenyl)propyl]-2-aminoindane;
(±)*cis*-1-[3-(4-Benzyloxyphenyl)propyl]-2-methylaminoindane;
(±)*trans*-1-[(4-Benzyl)benzyl]-2-aminoindane;
(±)*cis*-1-[(4-Benzyl)phenoxymethyl]-2-aminoindane;
(±)*cis*-1-[(4-Benzyl)phenoxymethyl]-2-methylaminoindane;
(±)*cis*-1-(4-Phenoxybenzyl)-2-methylaminoindane;
(±)*cis*-1-(3,4-Dichlorobenzyl)-2-aminoindane;
(±)*cis*-1-(3,4-Dichlorobenzyl)-2-methylaminoindane;
(±)*cis*-1-(4-Benzyloxybenzyl)-2-methylaminoindane;
(-)*cis*-1-(4-Benzyloxybenzyl)-2-methylamimoindane;
(±)*cis*-1-(4-Trifluoromethylbenzyl)-2-aminoindane;
(±)*cis*-1-(4-Phenoxybenzyl)-2-aminoindane;
(±)*cis*-[(4-Benzoyl)phenoxymethyl]-2-aminoindane;
(±)*cis*-1-(4-Benzyloxybenzyl)-2-dimethylaminoindane;
(±)*cis*-1-(4-Cyclopropylmethoxybenzyl)-2-aminoindane;
(±)*cis*-1-(3,4-Dichlorophenoxymethyl)-2-aminoindane;
(±)*cis*-1-(4-n-Butoxybenzyl)-2-aminoindane;
(±)*cis*-2-Amino-1-[(5-chloro-2-thienyl)methyl]indane;
(±)*cis*-1-[(5-Chloro-2-thienyl)methyl]-2-methylaminoindane;
(±)*cis*-2-Cyclopropylmethylamino-1-(3,4-dichlorobenzyl)-indane;
(±)*cis*-1-(3,4-Dichlorobenzyl)-2-dimethylaminoindane;
(±)*cis*-1-(3,4-Dichlorobenzyl)-2-isopropylaminoindane;
(±)*cis*-1-[4-(4-Fluorophenoxy)benzyl]-2-aminoindane;
(±)*cis*-1-[4-(4-Fluorophenoxy)benzyl]-2-methylaminoindane;
(±)*cis*-1-[4(4-Fluorophenoxy)benzyl]-2-isopropylaminoindane;
(±)*cis*-1-(4-Benzoylbenzyl)-2-aminoindane;
(±)*cis*-1-(4-Trifluoromethylbenzyl)-2-methylaminoindane;
or a salt thereof.

13. A process for the preparation of a compound of any of claims 1 to 12 which comprises :
(a) to prepare a compound of formula (I) wherein R² is methyl, reduction of a compound of formula (II) 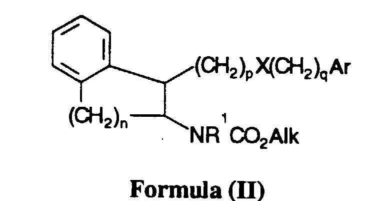 wherein n, p, q, R¹, Ar and X are as hereinbefore defined and Alk is a C₁₋₄alkyl group;
(b) to prepare a compound of formula (I) wherein at least one of R¹ and R² is hydrogen, deprotection of a compound of formula (II);
(c) to prepare a compound of formula (I) wherein R¹ and R² are both hydrogen and p is 1 to 4, reduction of a compound of formula (III): 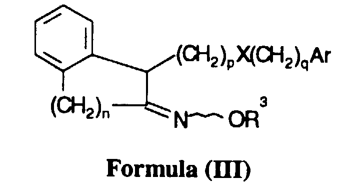 wherein n, p, q, X and Ar are as hereinbefore defined and R³ is hydrogen, C₁₋₄alkyl or phenylC₁₋₄alkyl (e.g. benzyl);
(d) to prepare a compound wherein X¹ is O, S or NH reaction of a compound of formula (IV) 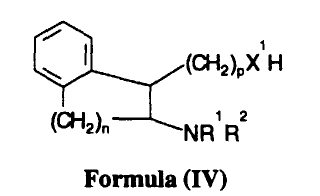 wherein R¹, R², p and n are as hereinbefore defined and X¹ is O, S or NH, with a compound of formula L(CH₂)_{q}Ar wherein L is a leaving group and q and Ar are as hereinbefore defined;
(e) to prepare a compound wherein X¹ is O, S or NH, reaction of a compound formula (V): 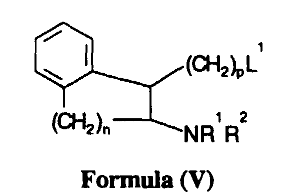 wherein R¹, R², p and n are as hereinbefore defined and L¹ is a group displaceable by a nucleophile, with a compound HX(CH₂)_{q}Ar wherein X, q and Ar are as hereinbefore defined;
(f) interconversion of a compound of formula (I) to a different compound of formula (I);
and optionally after any of the above processes, forming a salt of formula (I).

14. Use of a compound of any of claims 1 to 12 or a pharmaceutically acceptable salt thereof in the manufacture of a medicament for the treatment of a condition or disease related to the accumulation of calcium in the brain cells of a mammal.

15. A pharmaceutical composition comprising a compound of any of claims 1 to 12 or a pharmaceutically acceptable salt thereof and a pharmaceutically acceptable carrier or excipient.

## Patentansprüche

1. Verbindung der Formel (I): 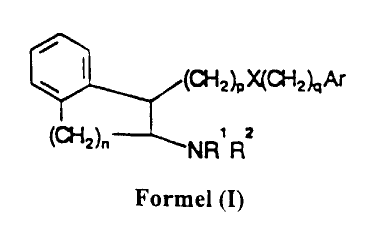 in der
X ein Sauerstoff-, Schwefelatom, eine NH-Gruppe oder eine Bindung bedeutet;
p und q unabhängig voneinander 0-4 sind, so daß die Summe von p+q mindestens 1 ist;
R¹ und R² jeweils unabhängig voneinander ein Wasserstoffatom, einen C₁₋₆Alkyl-, C₃₋₆Cycloalkyl- oder C₁₋₄-Alkyl-C₃₋₆-cycloalkylrest darstellen;
n 1, 2 oder 3 ist; und
Ar eine Phenylgruppe bedeutet, die gegebenenfalls mit 1 bis 3 Substituenten substituiert ist. ausgewählt aus: einem Halogenatom. C₁₋₄-Alkyl-, C₁₋₄-Alkoxy-, C₁₋₂-Alkylendioxyrest, einer Trifluormethyl-, Trifluormethoxy-, CN-, NO₂-, Aminogruppe, einem Mono- oder Dialkylaminorest, einer gegebenenfalls substituierten Benzoylgruppe und einem Rest der Formel Ph(CH₂)ᵣY(CH₂)ₛ-, wobei Ph eine gegebenenfalls substituierte Phenylgruppe darstellt, Y ein Sauerstoffatom oder eine Bindung bedeutet und r und s jeweils unabhängig voneinander 0-4 sind, mit der Maßgabe, daß die Summe von r+s nicht größer als 4 ist, oder
Ar ein gegebenenfalls substituiertes ungesättigtes monocyclisches Heteroarylringsystem. das 5 oder 6 Ringglieder enthält, oder ein gegebenenfalls substituiertes ungesättigtes oder teilweise gesättigtes bicyclisches Aryl- oder Heteroarylringsystem mit 8-10 Ringgliedem darstellt,
oder ein Salz davon.

2. Verbindung nach Anspruch 1, wobei Ar eine Phenylgruppe darstellt, die mit einem Rest der Formel Ph(CH₂)ᵣY(CH₂)ₛ- substituiert ist.

3. Verbindung nach Anspruch 2, wobei r und s unabhängig voneinander 0 oder 1 sind, so daß die Summe von r und s 1 nicht übersteigt.

4. Verbindung nach einem der Ansprüche 1 bis 3, wobei X eine Bindung bedeutet.

5. Verbindung nach einem der Ansprüche 1 bis 4, wobei R¹ eine Methyl- oder Isopropylgruppe darstellt.

6. Verbindung nach einem der Ansprüche 1 bis 5, wobei R² ein Wasserstoffatom bedeutet.

7. Verbindung nach Anspruch 4. wobei die Summe von p und q 1 bis 3 ist.

8. Verbindung nach einem der Ansprüche 1 bis 7, wobei n 1 ist.

9. Verbindung nach einem der Ansprüche 1 bis 8, wobei sich die gegebenenfalls substituierte Benzoylgruppe oder der Rest der Formel Ph(CH₂)ᵣY(CH₂)ₛ- in der 4-Stellung befindet.

10. Verbindung nach einem der Ansprüche 1 bis 9, wobei der Rest der Formel Ph(CH₂)ᵣY(CH₂)ₛ- eine gegebenenfalls substituierte Benzyloxy-, Benzyl- oder Phenoxygruppe darstellt. wobei die möglichen Substituenten ein Halogenatom. C₁₋₄-Alkoxy-, C₁₋₄Alkylrest, eine Trifluormethyl- oder Trifluormethoxygruppe sind.

11. Verbindung nach einem der Ansprüche 1 bis 10, wobei die Substituenten der gegebenenfalls substituierten Benzoyl-, Benzyloxy-, Benzyl- oder Phenoxygruppe ein 4-Fluor-, 4-Chlor-, 3-Fluor-, 3-Chlor- oder 3,4-Dichloratom sind.

12. Verbindung nach Anspruch 1, ausgewählt aus:
(±)*trans*-1-(4-Benzylbenzyl)-2-methylaminoindan;
(±)*trans*-1-(4-Benzylbenzyl)-2-dimethylaminoindan;
(±)*trans*-1-(4-Benzyloxybenzyl)-2-methylaminoindan;
(±)*trans*-1-(4-Benzyloxybenzyl)-2-dimethylaminoindan:
(±)*trans*-1-(4-Benzyloxybenzyl)-2-ethylaminoindan;
(±)*cis*-1-(4-Benzyloxybenzyl)-2-aminoindan;
(±)*cis*-1-(4-Benzyloxybenzyl)-2-methylaminoindan;
(±)*cis*-1-[3(4-Benzyloxyphenyl)propyl]-2-aminoindan;
(±)*cis*-1-[3-(4-Benzyloxyphenyl)propyl]-2-methylaminoindan;
(±)*trans*-1-[(4-Benzyl)benzyl]-2-aminoindan;
(±)*cis*-1-[(4-Benzyl)phenoxymethyl]-2-aminoindan;
(±)*cis*-1-[(4-Benzyl)phenoxymethyl]-2-methylaminoindan;
(±)*cis*-1-(4-Phenoxybenzyl)-2-methylaminoindan;
(±)*cis*-1-(3,4-Dichlorbenzyl)-2-aminoindan;
(±)*cis*-1-(3,4-Dichlorbenzyl)-2-methylaminoindan;
(+)*cis*-1-(4-Benzyloxybenzyl)-2-methylaminoindan;
(-)*cis*-1-(4-Benzyloxybenzyl)-2-methylarninoindan;
(±)*cis*-1-(4-Trifluormethylbenzyl)-2-aminoindan;
(±)*cis*-1-(4-Phenoxybenzyl)-2-aminoindan;
(±)*cis*-1-[(4-Benzoyl)phenoxymethyl]-2-aminoindan;
(±)*cis*-1-(4-Benzyloxybenzyl)-2-dimethylaminoindan;
(±)*cis*-1-(4-Cyclopropylmethoxybenzyl)-2-aminoindan;
(±)*cis*-1-(3,4-Dichlorphenoxymethyl)-2-aminoindan;
(±)*cis*-1-(4-n-Butoxybenzyl)-2-aminoindan;
(±)*cis*-2-Amino-1-[(5-chlor-2-thienyl)methyl]indan;
(±)*cis*-1-[(5-Chlor-2-thienyl)methyl]-2-methylaminoindan;
(±)*cis*-2-Cyclopropylmethylamino-1-(3,4-dichlorbenzyl)indan;
(±)*cis*-1-(3,4-Dichlorbenzyl)-2-dimethylaminoindan;
(±)*cis*-1-(3,4-Dichlorbenzyl)-2-isopropylaminoindan;
(±)*cis*-1-[4-(4-Fluorphenoxy)benzyl]-2-aminoindan;
(±)*cis*-1-[4-(4-Fluorphenoxy)benzyl]-2-methylaminoindan;
(±)*cis*-1-[4-(4-Fluorphenoxy)benzyl]-2-isopropylaminoindan;
(±)*cis*-1-(4-Benzoylbenzyl)-2-aminoindan;
(±)*cis*-1-(4-Trifluormethylbenzyl)-2-methylaminoindan;
oder ein Salz davon.

13. Verfahren zur Herstellung einer Verbindung nach einem der Ansprüche 1 bis 12, das umfaßt:
(a) zur Herstellung einer Verbindung der Formel (I), in der R² eine Methylgruppe bedeutet, Reduktion einer Verbindung der Formel (II) 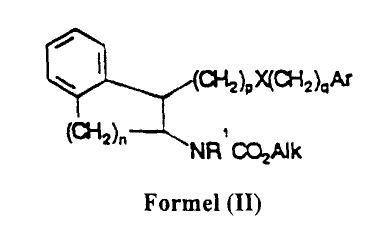 in der n, p, q, R¹, Ar und X wie vorstehend definiert sind und Alk einen C₁₋₄-Alkylrest darstellt;
(b) zur Herstellung einer Verbindung der Formel (I), in der mindestens einer der Reste R¹ und R² ein Wasserstoffatom bedeutet, Abspaltung der Schutzgruppe von einer Verbindung der Formel (II);
(c) zur Herstellung einer Verbindung der Formel (I), in der R¹ und R² beide ein Wasserstoffatom darstellen und p 1 bis 4 ist, Reduktion einer Verbindung der Formel (III): 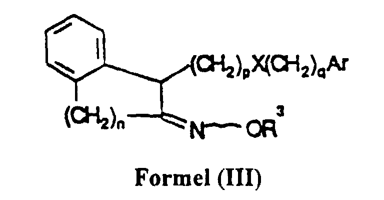 in der n, p, q, X und Ar wie vorstehend definiert sind und R³ ein Wasserstoffatom, einen C₁₋₄-Alkylrest oder Phenyl-C₁₋₄-alkylrest (z.B. eine Benzylgruppe) bedeutet;
(d) zur Herstellung einer Verbindung der Formel (I), in der X ein Sauerstoff-, Schwefelatom oder eine NH-Gruppe darstellt, Reaktion einer Verbindung der Formel (IV) 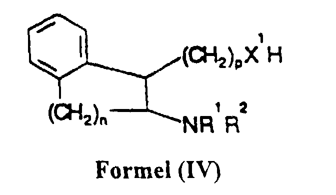 in der R¹, R², p und n wie vorstehend definiert sind und X¹ ein Sauerstoff-, Schwefelatom oder eine NH-Gruppe bedeutet, mit einer Verbindung der Formel L(CH₂)_{q}Ar, in der L eine Abgangsgruppe darstellt und q und Ar wie vorstehend definiert sind;
(e) zur Herstellung einer Verbindung der Formel (I), in der X ein Sauerstoff- Schwefelatom oder eine NH-Gruppe bedeutet, Reaktion einer Verbindung der Formel (V): 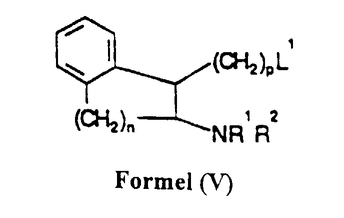 in der R¹, R², p und n wie vorstehend definiert sind und L¹ eine durch ein Nucleophil ersetzbare Gruppe darstellt, mit einer Verbindung HX(CH₂)_{q}Ar, in der X, q und Ar wie vorstehend definiert sind;
(f) wechselseitige Umwandlung einer Verbindung der Formel (I) in eine unterschiedliche Verbindung der Formel (I)
und gegebenenfalls im Anschluß an irgend eines der vorstehenden Verfahren Erzeugung eines Salzes der Formel (I).

14. Verwendung einer Verbindung nach einem der Ansprüche 1 bis 12 oder eines pharmazeutisch verträglichen Salzes davon bei der Herstellung eines Arzneimittels zur Behandlung eines Zustandes oder einer Krankheit, die mit der Anhäufung von Calcium in den Hirnzellen eines Säugers verbunden ist.

15. Arzneimittel, umfassend eine Verbindung nach einem der Ansprüche 1 bis 12 oder ein pharmazeutisch verträgliches Salz davon und einen pharmazeutisch verträglichen Träger oder Excipienten.

## Revendications

1. Composé de formule (I) : 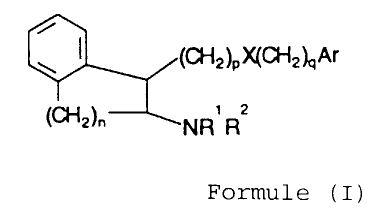 dans laquelle
x représente O, S, un groupe NH ou une liaison ;
p et q ont indépendamment une valeur de O à 4 de telle sorte que la somme p+q soit au moins égale à 1 ;
R¹ et R² représentent chacun indépendamment l'hydrogène, un groupe alkyle en C₁ à C₆, cycloalkyle en C₃ à C₆ ou (alkyle en C₁ à C₄)-(cyloalkyle en C₃ à C₆) ;
n est égal à 1, 2 ou 3 ; et
Ar représente un groupe phényle facultativement substitué avec 1 à 3 substituants choisis entre des substituants : halogéno, alkyle en C₁ à C₄, alkoxy en C₁ à C₄, alkylenedioxy en C₁ ou C₂, trifluorométhyle, trifluorométhoxy, CN, NO₂, amino, mono- ou di-alkylamino, benzoyle facultativement substitué et Ph(CH₂)ᵣY(CH₂)ₛ- dans lequel Ph représente un groupe phényle facultativement substitué, Y représente l'oxygène ou une liaison et r et s ont chacun indépendamment une valeur de O à 4 sous réserve que la somme r+s ne soit pas supérieure à 4, ou
Ar représente un système cyclique hétéroaryle monocyclique insaturé facultativement substitué dont le noyau contient 5 ou 6 atomes, ou un système cyclique aryle ou hétéroaryle bicyclique insaturé ou partiellement saturé, facultativement substitué, contenant 8 à 10 atomes dans le système cyclique,
ou un de ses sels.

2. Composé suivant la revendication 1, dans lequel Ar représente un groupe phényle substitué avec un groupe Ph(CH₂)ᵣY(CH₂)ₛ-.

3. Composé suivant la revendication 2, dans lequel r et s ont indépendamment la valeur 0 ou 1, de telle sorte que la somme de r et s n'excède pas 1.

4. Composé suivant l'une quelconque des revendications 1 à 3, dans lequel X représente une liaison.

5. Composé suivant l'une quelconque des revendications 1 à 4, dans lequel R¹ représente un groupe méthyle ou isopropyle.

6. Composé suivant l'une quelconque des revendications 1 à 5, dans lequel R² représente l'hydrogène.

7. Composé suivant la revendication 4, dans lequel la somme de p et q a une valeur de 1 à 3.

8. Composé suivant l'une quelconque des revendications 1 à 7, dans lequel n est égal à 1.

9. Composé suivant l'une quelconque des revendications 1 à 8, dans lequel le groupe benzoyle facultativement substitué ou le groupe Ph(CH₂)ᵣY(CH₂)ₛ- est en position 4.

10. Composé suivant l'une quelconque des revendications 1 à 9, dans lequel Ph(CH₂)ᵣY(CH₂)ₛ- est un groupe benzyloxy, benzyle ou phénoxy facultativement substitué, dans lequel les substituants facultatifs sont des substituants halogéno, alkoxy en C₁ à C₄, alkyle en C₁ à C₄, trifluorométhyle ou trifluorométhoxy.

11. Composé suivant l'une quelconque des revendications 1 à 10, dans lequel les substituants sur le groupe benzoyle, benzyloxy, benzyle ou phénoxy facultativement substitué sont des substituants 4-fluoro, 4-chloro, 3-fluoro, 3-chloro ou 3,4-dichloro.

12. Composé suivant la revendication 1, choisi entre les suivants :
(±)trans-1-(4-benzylbenzyl)-2-méthylaminoindane ;
(±)trans-1-(4-benzylbenzyl)-2-diméthylaminoindane ;
(±)trans-1-(4-benzyloxybenzyl)-2-méthylaminoindane ;
(±)trans-1-(4-benzyloxybenzyl)-2-diméthylaminoindane ;
(±)trans-1-(4-benzyloxybenzyl)-2-éthylaminoindane ;
(±)cis-1-(4-benzyloxybenzyl)-2-aminoindane ;
(±)cis-1-(4-benzyloxybenzyl)-2-méthylaminoindane ;
(±)cis-1-[3-(4-benzyloxyphényl)propyl]-2-aminoindane ;
(±)cis-1-[3-(4-benzyloxyphényl)propyl]-2-méthylaminoindane ;
(±)trans-1-[(4-benzyl)benzyl]-2-aminoindane ;
(±)cis-1-[(4-benzyl)phénoxyméthyl]-2-aminoindane ;
(±)cis-1-[(4-benzyl)phénoxyméthyl]-2-méthylaminoindane ;
(±)cis-1-(4-phénoxybenzyl)-2-méthylaminoindane ;
(±)cis-1-(3,4-dichlorobenzyl)-2-aminoindane ;
(±)cis-1-(3,4-dichlorobenzyl)-2-méthylaminoindane ;
(±)cis-1-(4-benzyloxybenzyl)-2-méthylaminoindane ;
(-)cis-1-(4-benzyloxybenzyl)-2-méthylaminoindane ;
(±)cis-1-(4-trifluorométhylbenzyl)-2-aminoindane ;
(±)-cis-1-(4-phénoxybenzyl)-2-aminoindane ;
(±)cis-1-[(4-benzoyl)phénoxyméthyl]-2-aminoindane ;
(±)cis-1-(4-benzyloxybenzyl)-2-diméthylaminoindane ;
(±)cis-1-(4-cyclopropylméthoxybenzyl)-2-aminoindane ;
(±)cis-1-(3-dichlorophénoxyméthyl)-2-aminoindane ;
(±)cis-1-(4-n-butoxybenzyl)-2-aminoindane ;
(±)cis-2-amino-1-[(5-chloro-2-thiényl)méthyl]indane ;
(±)cis-1-[(5-chloro-2-thiényl)méthyl]-2-méthylaminoindane ;
(±)cis-2-cyclopropylméthylamino-1-(3,4-dichlorobenzyl)indane ;
(±) cis-1-(3,4-dichlorobenzyl)-2-diméthylaminoindane ;
(±) cis-1-(3,4-dichlorobenzyl)-2-isopropylaminoindane ;
(±) cis-1-[4-(4-fluorophénoxy)benzyl]-2-aminoindane ;
(±) cis-1-[4-(4-fluorophénoxy)benzyl]-2-méthylaminoindane ;
(±) cis-1-[4-(4-fluorophénoxy)benzyl]-2-isopropylaminoindane ;
(±)cis-1-(4-benzoylbenzyl)-2-aminoindane;
(±) cis-1-(4-trifluorométhylbenzyl)-2-méthylaminoindane ; ou un de ses sels.

13. Procédé pour la préparation d'un composé suivant l'une quelconque des revendications 1 à 12, qui comprend :
(a) pour la préparation d'un composé de formule (I) dans laquelle R² représente un groupe méthyle, la réduction d'un composé de formule (II) 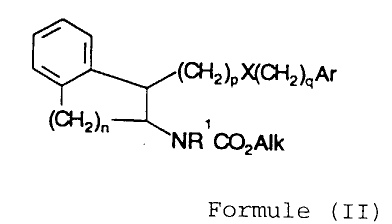 dans laquelle n, p, q, R¹, Ar et X répondent aux définitions précitées et Alk représente un groupe alkyle en C₁ à C₄ ;
(b) pour la préparation d'un composé de formule (I) dans laquelle au moins un des groupes R¹ et R² représente l'hydrogène, la suppression de la protection d'un composé de formule (II) ;
(c) pour la préparation d'un composé de formule (I) dans laquelle R¹ et R² représentent l'un et l'autre l'hydrogène et p a une valeur de 1 à 4, la réduction d'un composé de formule (III) : 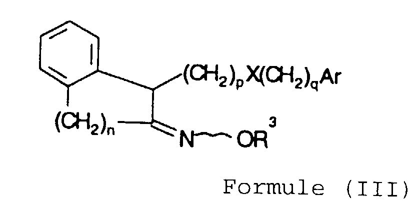 dans laquelle n, p, q, X et Ar répondent aux définitions précitées et R³ représente l'hydrogène, un groupe alkyle en C₁ à C₄ ou phényl-(alkyle en C₁ à C₄)(par exemple benzyle) ;
(d) pour la préparation d'un composé dans lequel X¹ représente O, S ou un groupe NH, la réaction d'un composé de formule (IV) 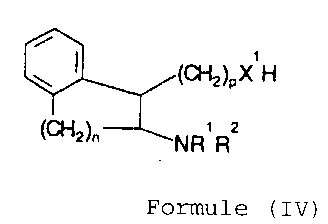 dans laquelle R¹, R², p et n répondent aux définitions précitées et X¹ représente O, S ou un groupe NH, avec un composé de formule L(CH₂)_{q}Ar dans laquelle L représente un groupe partant et q et Ar répondent aux définitions précitées ;
(e) pour la préparation d'un composé dans lequel X¹ représente O, S ou un groupe NH, la réaction d'un composé de formule (V) : 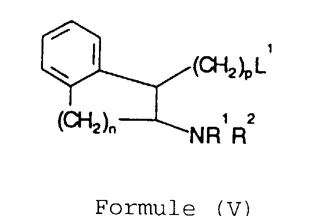 dans laquelle R¹, R², p et n répondent aux définitions précitées et L¹ représente un groupe déplaçable par un agent nucléophile, avec un composé de formule HX(CH₂)_{q}Ar dans laquelle X, q et Ar répondent aux définitions précitées ;
(f) l'interconversion d'un composé de formule (I) en un composé différent de formule (I) ;
et facultativement, après n'importe lequel des procédés précités, la formation d'un sel du composé de formule (I).

14. Utilisation d'un composé suivant l'une quelconque des revendications 1 à 12 ou d'un de ses sels pharmaceutiquement acceptables dans la production d'un médicament destiné au traitement d'une affection ou maladie en rapport avec l'accumulation de calcium dans les cellules cérébrales d'un mammifère.

15. Composition pharmaceutique comprenant un composé suivant l'une quelconque des revendications 1 à 12 ou un de ses sels pharmaceutiquement acceptables et un support ou excipient pharmaceutiquement acceptable.
